# FASCICULE DE BREVET EUROPEEN

(11) **EP 0 993 616 B1**
(45) Date de publication et mention de la délivrance du brevet: **19.05.2004**
(21) Numéro de dépôt: 98935098.8
(22) Date de dépôt: 03.07.1998
(51) Int. Cl.: G01N 33/569, G01N 33/536, G01N 33/68, C07K 14/05

(54) **REACTIF DE DETECTION ET SUIVI DES INFECTIONS VIRALES**
REAGENZ ZUM NACHWEIS UND WEITERVERFOLGEN VON VIRUSINFEKTIONEN
REAGENT FOR DETECTING AND MONITORING VIRAL INFECTIONS

(30) Priorité: 04.07.1997 FR 9708537
(43) Date de publication de la demande: 19.04.2000
(73) Titulaire: INSTITUT PASTEUR DE LILLE, 59019 Lille Cédex (FR); CENTRE NATIONAL DE LA RECHERCHE SCIENTIFIQUE, 75016 Paris Cédex (FR)
(72) Inventeur: TRANCHAND BUNEL, Denis, F-59790 Ronchin (FR); GRAS MASSE, Hélène, F-59710 Merignies (FR); AURIAULT, Claude, F-59310 Nomain (FR); TARTAR, André, F-62490 Vitry-en-Artois (FR); DIESIS, Eric, F-59320 Haubourdin (FR); BOUREZ, Brigitte, F-59115 Leers (FR)
(74) Mandataire: Vialle-Presles, Marie José
(86) Numéro de dépôt international: PCT/FR1998/001432
(87) Numéro de publication internationale: WO 1999/001767

(56) Documents cités:
- EP-A- 0 442 394
- EP-A- 0 508 427
- EP-A- 0 574 048
- EP-A- 0 649 904
- EP-A- 0 754 755
- WO-A-93/18054
- WO-A-96/00784
- WO-A-96/30547
- VAN GRUNSVEN W M J ET AL: "Localization and diagnostic application of immunodominant domains of the BFRF3-encoded Epstein - Barr virus capsid protein." JOURNAL OF INFECTIOUS DISEASES 170 (1). 1994. 13-19. ISSN: 0022-1899, XP002058643 cité dans la demande
- HSU T-Y ET AL: "USE OF ANTIGEN EXPRESSED IN BACTERIA FOR DETECTION OF EBV -SPECIFIC THYMIDINE KINASE ANTIBODIES IN SERA FROM PATIENTS WITH NASOPHARYNGEAL CARCINOMA." J MED VIROL 38 (3). 1992. 214-219. CODEN: JMVIDB ISSN: 0146-6615, XP002058644
- AURIAULT C ET AL: "Immunological properties of synthetic peptide constructs from HIV and the parasite Schistosoma mansoni: Expertise in an immunoprophylactic strategy." JOINT MEETING OF THE AMERICAN ACADEMY OF ALLERGY, ASTHMA AND IMMUNOLOGY, THE AMERICAN ASSOCIATION OF IMMUNOLOGISTS AND THE CLINICAL IMMUNOLOGY SOCIETY, SAN FRANCISCO, CALIFORNIA, USA, FEBRUARY 21-26, 1997. JOURNAL OF ALLERGY AND CLINICAL IMMUNOLOGY 9, XP002058645

## Description

La présente invention est relative à un réactif de détection et de suivi des infections virales, telles que celles provoquées par le virus d'Epstein-Barr ou EBV, qui est, notamment, l'agent causatif de la mononucléose infectieuse, par le virus de l'hépatite C (HCV) ou par le virus de l'immunodéficience humaine (HIV) et à ses applications pour la détection d'une infection virale, notamment d'une infection à EBV, à n'importe quel stade de l'infection (primo-infection, porteurs sains et tumeurs induites).

L'EBV est un herpèsvirus, qui infecte de préférence les lymphocytes B et les cellules épithéliales.

Ce virus lymphocryptique échappe à la surveillance immunologique. En général, le virus est porté par des porteurs sains, sans symptômes particuliers ; cependant, dans des conditions d'immunodéficience, telles que le SIDA ou une chimiothérapie après transplantation, ainsi que dans les zones endémiques, telles qu'en Afrique ou en Asie, le potentiel oncogène de l'EBV est libéré et conduit à l'émergence de différentes tumeurs, telles que le lymphome africain de Burkitt, le carcinome indifférencié du nasopharynx, certains lymphomes et la maladie de Hodgkin.

A l'heure actuelle, le sérodiagnostic de l'EBV est effectué, soit à l'aide de la réaction de Paul-Bunnell (détection d'anticorps hétérophiles), méthode simple et rapide mais non spécifique (LINDERHOLM M. et al., *J. Clin. Microbiol.,* 1994, 32, 1, 259-261), soit à l'aide de tests d'immunofluorescence (IF), spécifiques pour chaque classe d'antigènes (VCA, EA et EBNA).

Le développement d'un test de diagnostic plus simple et moins cher, mettant en oeuvre une méthode immunoenzymatique, telle que la méthode ELISA, est, dans ce contexte, particulièrement souhaitable et plusieurs tests ont ainsi été proposés (M. GORGIEVSKY-HRISOHO et al., *J. Clin. Microbiol.,* 1990, **28**, 2305-2311 ; JM. MIDDELDORP et al., *J. Virol. Methods,* 1988, **21**, 133-159).

Les méthodes immunoenzymatiques proposées dans l'Art antérieur présentent, pour la plupart, l'inconvénient majeur de manquer de sensibilité de détection (M. GORGIEVSKY-HRISOHO et al. précité ; JM. MIDDELDORP et al. précité). Pour résoudre ce problème du manque de sensibilité, certains Auteurs, ont proposés d'utiliser, en combinaison, différents antigènes; de telles combinaisons augmentent la sensibilité du test dans lequel ces combinaisons sont mises en oeuvre, mais pas la spécificité. Les combinaisons d'antigènes qui ont été proposées dans l'Art antérieur comprennent, par exemple, l'antigène p47-52 (antigène majeur EA-D, codé par le cadre de lecture ouvert dénommé BMRF1, n° d'accès SWISS-PROT P03191), l'antigène p38 (codé par le cadre de lecture ouvert dénommé BALF2, n° d'accès SWISS-PROT P03227) et l'antigène gp125 ou gp110 (codé par le cadre de lecture ouvert dénommé BALF4, n° d'accès SWISS-PROT P03188) (W.M.J. Van GRUNSVEN et al., *J. Virol.,* 1993, 67, 3908-3916).

Plus récemment, il a été montré qu'un antigène de capside de 18 kDa (VCAp18), codé par le cadre de lecture ouvert BFRF3 (n° d'accès SWISS-PROT P14348), est reconnu par les porteurs sains de l'EBV dans l'analyse par *immunoblot* et semble ne pas présenter de séquences homologues avec les autres herpès virus humains, et ce, contrairement aux protéines majeures, telle que le VCAp40 (séquence codée par le cadre de lecture ouvert dénommé BdRF1, n° d'accès SWISS-PROT P03219) et le gp125/110 (R. BAER et al., *Nature,* 1984, **310**, 207-211 ; M.S. CHEE et al., *Curr. Topics Microbiol. Immunol.,* 1990,**154**, 125-170 ; A.J. DAVIDSON, *J. Gen. Virol.,* 1986, **67**, 1759-1816). La carte des domaines antigéniques de l'antigène VCAp18 a été réalisée et le domaine antigénique majeur a été localisé dans la région C-terminale de la protéine (W.M.J. Van GRUNSVEN et al.. *J. Infect. Dis.,* 1994, **170**, 13-18).

Toutefois, les tests ELISA effectués avec cet antigène de capside VCAp18 ont l'inconvénient :
- de ne pas présenter une sensibilité et une spécificité suffisante pour détecter l'ensemble des Ig anti-VCA produites (faux-positifs et/ou faux-négatifs) ; en particulier, en ce qui concerne les faux-négatifs, ils sont essentiellement dus à la faible taille du peptide synthétique VCAp18/SEQ ID n°2 (24 acides aminés, SEQ ID n°2 de la liste des séquences), qui peut, en conséquence, ne pas être reconnu par tous les individus VCA-positifs et/ou en raison de l'hétérogénéité de la réactivité des anticorps vis-à-vis du peptide synthétique, par rapport au même peptide, dans son environnement naturel.
- de ne pas permettre le diagnostic différentiel des différents stades de l'infection à EBV et/ou des pathologies induites par l'EBV, en fonction du profil isotypique des Ig produites (IgG, IgA et IgM), et ce, malgré l'utilisation du fragment C-terminal de la protéine VCAp18 (SEQ ID n°1 de la liste des séquences), comme réactif.

Une situation similaire se rencontre avec d'autres virus, tels que HCV ou HIV ; en effet, de manière générale, l'utilisation d'un seul ou de plusieurs fragments immunodominants ne présente pas une sensibilité suffisante pour éviter les faux-négatifs.

C'est pourquoi la Demanderesse s'est donné pour but de pourvoir à un nouveau réactif de détection des infections virales, apte à être utilisé dans les tests immunoenzymatiques, qui soit à la fois spécifique et sensible et qui permette d'obtenir un gain de sensibilité d'au moins 15 à 30 % par rapport aux réactifs de l'art antérieur.

C'est également pourquoi la Demanderesse s'est donné pour but de pourvoir à un nouveau réactif de détection des infections à EBV, apte à être utilisé dans les tests immunoenzymatiques, qui soit à la fois spécifique et sensible et qui permette un diagnostic différentiel du stade de l'infection et/ou de la pathologie, en fonction de l'isotype prévalent ; en effet, la présence d'IgM humaines anti-VCA est essentiellement le signe d'une primo-infection, la présence d'IgG humaines anti-VCA est essentiellement le signe d'une infection passée (porteurs sains, généralement), alors que la présence d'IgA humaines anti-VCA suggère l'émergence d'une tumeur. Un tel réactif répond mieux aux besoins de la pratique que les réactifs de l'Art antérieur, dans le cadre des tests immunoenzymatiques, notamment de type ELISA.

La présente invention a pour objet un réactif de diagnostic d'une infection provoquée par un virus, caractérisé en ce qu'il comprend essentiellement un mélange constitué par (1) un fragment immunodominant d'une protéine dudit virus comprenant au plus 60 acides aminés, de préférence entre 20 et 30 acides aminés et (2) un mélange (dénommé mixotope) de peptides combinatoires convergents, dérivés dudit fragment immmunodominant, lesquels peptides sont obtenus par dégénération artificielle complète ou partielle dudit fragment immunodominant par remplacement systématique ou partiel de chaque amino acide par un autre selon une matrice de remplaçabilité convenable.

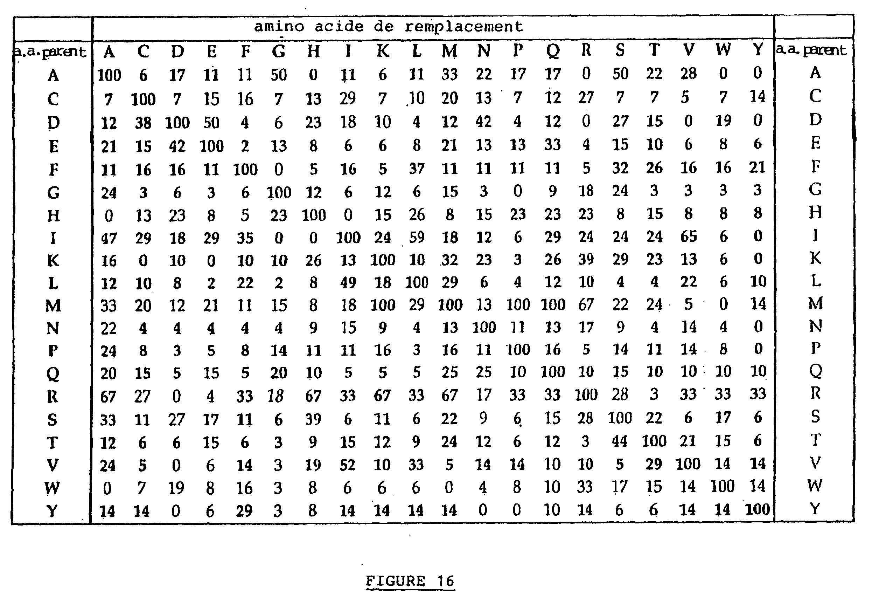

Au sens de l'invention, on entend par mixotope, le mélange de tous les peptides combinatoires, obtenus à partir du fragment immunodominant sélectionné, par dégénération artificielle ou construite ; ils sont, de préférence, obtenus au cours d'une synthèse unique et représentent l'antigène peptidique et sa variabilité, dans sa fonction de reconnaissance d'une population d'anticorps ; différents mixotopes peuvent être obtenus à partir du même peptide ; les facteurs qui interviennent dans la constitution d'un mixotope sont :
- d'une part, le pourcentage de dégénération du fragment immunodominant natif sélectionné (dégénération totale ou partielle) ; les acides aminés conservés (isolés ou formant une séquence), dans le cas d'une dégénération partielle, sont, de préférence, pour ce qui concerne l'EBV, ceux qui interviennent dans la structuration de la protéine VCAp18 et
- d'autre part, le mode de sélection de la substitution des acides aminés dudit fragment immunodominant natif ; pour chaque position de la séquence du fragment immunodominant natif choisi, la substitution en acides aminés est sélectionnée sur la base de la matrice de remplacement, établie par H.M. GEYSEN et al. *(J. Mol. Recog.,* 1988, 1, 32-41), ou modifiée, comme illustrée à la figure 16, en tenant compte de la tolérance de la reconnaissance d'anticorps, en fonction de la substitution en aminoacides dans les épitopes linéaires : on choisit de préférence, pour une position donnée, les acides aminés présentant le pourcentage de « remplaçabilité » le plus élevé. Toutefois, il est préférable de prendre en compte la conformation des épitopes naturels, avant dégénération.

Le mixotope au sens de la présente invention, constitué de peptides combinatoires convergents, dérivés d'un fragment immunodominant natif, représente donc une dégénérescence artificielle et non naturelle de la structure native par le remplacement systématique ou partiel de chaque aminoacide par un autre issu de la matrice de remplaçabilité de GEYSEN ou de la matrice selon la figure 16.

Également au sens de l'invention, on entend par matrice de remplaçabilité construite, une matrice qui ne reproduit par les variations naturelles ou observées fréquemment dans l'évolution ; la matrice de remplaçabilité de GEYSEN ou la matrice selon la figure 16, sont particulièrement adaptées.

De manière surprenante, les réactifs selon l'invention permettent d'obtenir des résultats fiables, reproductibles, très sensibles et très spécifiques, dans la mesure où les combinaisons selon l'invention présentent un effet de synergie dans la détection des anticorps induits par les virus ; en particulier, on obtient de manière générale, quel que soit le virus (HIV, HCV, EBV, par exemple), un gain de sensibilité de 15 à 30 %.

La présente invention a également pour objet un réactif de diagnostic selon l'invention, caractérisé en ce que pour le diagnostic des infections à EBV, il comprend essentiellement un mélange constitué par (1) un fragment C-terminal de l'antigène de capside viral de 18 kDa (protéine VCAp18, SEQ ID n°1) du virus d'Epstein-Barr (EBV) comprenant au plus 60 acides aminés, de préférence entre 20 et 30 acides aminés et (2) un mélange (dénommé mixotope) de peptides combinatoires convergents, dérivés dudit fragment C-terminal ou peptide VCAp18.

Selon un mode de réalisation avantageux du réactif selon l'invention, ledit fragment C-terminal de la protéine VCAp18 est sélectionné dans le groupe constitué par les peptides VCAp18/SEQ ID n°2, VCAp18/SEQ ID n°3, VCAp18/SEQ ID n°4 et VCAp18/SEQ ID n°5.

De manière inattendue, la combinaison d'un fragment C-terminal de la protéine VCAp18 avec un mixotope, dérivé dudit fragment, et ce quelle que soit la configuration du fragment C-terminal choisi, améliore, de manière significative, la sensibilité et la spécificité du sérodiagnostic immunoenzymatique de l'EBV; en effet, si on compare un test ELISA mettant en oeuvre un réactif selon l'invention tel que défini ci-dessus, avec un test ELISA, mettant en oeuvre uniquement un peptide VCAp18, on obtient avec le réactif selon l'invention, effectivement une sensibilité de 100 % et une spécificité de 100 %, du fait de la liaison de l'ensemble des Ig (G, A et M) anti-VCA audit réactif.

De manière surprenante, les réactifs selon l'invention permettent d'obtenir des résultats fiables, reproductibles, très sensibles et très spécifiques, du fait que les combinaisons selon l'invention présentent un effet de synergie dans la détection de l'ensemble des Ig humaines anti-VCA produites.

Le mélange de peptides dégénérés, artificiellement produit, au cours d'une même synthèse est combiné avec le peptide natif. Une telle combinaison permet, de manière inattendue, d'augmenter la réactivité du mélange d'antigènes produit vis-à-vis des anticorps naturellement induits par le virus.

Dans ces conditions, il est possible d'obtenir des sensibilités de l'ordre de 100 % et de 97 % respectivement dans les populations de post-infectés et de primo-infectés, tout en conservant dans tous les tests réalisés 100 % de spécificité.

Par exemple :
* pour le peptide VCAn18/SEQ ID n°2, on peut ainsi obtenir les trois mixotopes suivants :
   - le mixotope correspondant à une dégénération de l'ensemble de la séquence du peptide VCAp18/SEQ ID n°2, dénommé ci-après mixotope MIXO,
   - le mixotope, dans lequel les résidus proline du peptide VCAp18/SEQ ID n°2 sont préférentiellement conservés, cette dernière intervenant notamment dans la conformation; ledit mixotope, dénommé ci-après mixotope MIXO(P), correspond à une dégénération partielle de la séquence du peptide VCAp18/SEQ ID n°2 et
   - le mixotope, dans lequel à la fois les résidus proline et la séquence Gly Ser Gly Gly Gly Gly (SEQ ID n°6) du peptide VCAp18/SEQ ID n°2 sont préférentiellement conservés ; ledit mixotope, dénommé ci-après mixotope MIXO(P,G), correspond à une dégénération partielle de la séquence du peptide VCAp18/SEQ ID n°2.
* pour le peptide VCAp18/SEQ ID n°3, on peut ainsi obtenir les mixotopes suivants :
   - le mixotope correspondant à une dégénération de l'ensemble de la séquence du peptide VCAp18/SEQ ID n°3,
   - le mixotope, dans lequel le résidu proline et/ou les résidus sérine de séquence répétitive du peptide VCAp18/SEQ ID n°3 sont préférentiellement, conservés ; ledit mixotope correspond à une dégénération partielle de la séquence du peptide VCAp18/SEQ ID n°3,
   - le mixotope, dans lequel le résidu proline et les résidus alanine de séquence répétitive du peptide VCAp18/SEQ ID n°3 sont préférentiellement conservés ; ledit mixotope correspond à une dégénération partielle de la séquence du peptide VCAp18/SEQ ID n°3 et
   - le mixotope, dans lequel à la fois le résidu proline, les résidus serine et les résidus alanine du peptide VCAp18/SEQ ID n°3 sont préférentiellement conservés.
* pour le peptide VCAp18/SEQ ID n°4, on peut ainsi obtenir les mixotopes suivants :
   - le mixotope correspondant à une dégénération de l'ensemble de la séquence du peptide VCAp18/SEQ ID n°4,
   - le mixotope, dans lequel les résidus sérine, proline et/ou alanine et/ou la séquence Gly ser Gly Gly Gly Gly (SEQ ID n°6) et/ou la séquence Ala Ala Ala Ser Ala Ala Ala Ala (SEQ ID n°7) du peptide VCAp18/SEQ ID n°4 sont préférentiellement conservés ; ledit mixotope correspond à une dégénération partielle de la séquence du peptide VCAp 18/SEQ ID n°4.
* pour le peptide VCAp18/SEQ ID n°5, on peut ainsi obtenir les mixotopes suivants :
   - le mixotope correspondant à une dégénération de l'ensemble de la séquence du peptide VCAp18/SEQ ID n°5,
   - le mixotope, dans lequel les résidus sérine et/ou alanine du peptide VCAp18/SEQ ID n°5 sont préférentiellement conservés ; ledit mixotope correspond à une dégénération partielle de la séquence du peptide VCAp18/SEQ ID n°5,
   - le mixotope, dans lequel à la fois les résidus serine et la séquence Ala Ala Ala Ser Ala Ala Ala Ala (SEQ ID n°7) du peptide VCAp18/SEQ ID n°5 sont préférentiellement conservés ; ledit mixotope correspond à une dégénération partielle de la séquence du peptide VCAp18/SEQ ID n°5.

Les différents mixotopes correspondant à une dégénération partielle du peptide VCAp18 choisi, conservent une séquence native qui, de préférence, intervient dans la structuration de la protéine VCAp18.

Selon un autre mode de réalisation avantageux du réactif selon l'invention, il est fixé sur un support solide, de préférence des plaques de microtitration.

Selon un autre mode de réalisation avantageux du réactif selon l'invention, le rapport peptide C-terminal:mixotope dans le mélange est compris entre 1:10 et 1:100.

La présente invention a également pour objet un procédé de diagnostic d'une infection virale, par une méthode immuno-enzymatique, caractérisé en ce qu'il met en oeuvre un réactif de diagnostic selon l'invention.

En particulier, pour ce qui concerne les infections à EBV, la présente invention a également pour objet un procédé de diagnostic par une méthode immuno-enzymatique, caractérisé en ce qu'il comprend :
- la mise en contact d'un sérum à analyser avec un réactif tel que défini ci-dessus,
- l'addition d'anticorps anti-Ig humaines, couplés à une enzyme et
- la révélation qualitative et/ou quantitative des anticorps anti-VCA éventuellement présents dans le sérum à analyser par addition du substrat de l'enzyme.

Selon un mode de mise en oeuvre avantageux dudit procédé, il comprend:
- la fixation d'un réactif selon l'invention sur un support, tel qu'une plaque de microtitration,
- l'addition du sérum à analyser et
- la détection de la fixation des anticorps anti-VCA présents dans ledit sérum par addition d'anticorps anti-Ig humaines (G-A-M), couplés à une enzyme et
- la révélation qualitative et/ou quantitative au spectrophotomètre par addition du substrat de l'enzyme.

La présente invention a également pour objet un procédé de surveillance et de détection différentielle des stades d'une infection à EBV, par une méthode immuno-enzymatique, caractérisé en ce qu'il comprend :
- la fixation d'un réactif selon l'invention sur un support, tel qu'une microplaque de titration,
- l'addition du sérum à analyser,
- la détection de la fixation des anticorps anti-VCA, éventuellement présents dans ledit sérum, par addition d'anticorps anti-Ig humaines couplés à une enzyme, lesquels anticorps sont sélectionnés dans le groupe constitué par les anticorps anti-IgG humaines, les anticorps anti-IgM humaines et les anticorps anti-IgA humaines et
- la révélation qualitative ou quantitative au spectrophotomètre par addition du substrat de l'enzyme.

La primo-infection induit la formation d'anticorps dirigés contre les différents antigènes de l'EBV ; il s'agit en particulier des anticorps dirigés contre l'antigène de capside virale (VCA), des anticorps dirigés contre l'antigène nucléaire (EBNA), des anticorps dirigés contre l'antigène précoce (EA) et des anticorps dirigés contre l'antigène de membrane (MA).

Ces différents anticorps n'apparaissent pas au même stade de l'infection ; en particulier, les Ig anti-VCA sont produites très précocement et restent présentes pendant toute la durée de vie de l'hôte. Ceci implique que la détection des IgM et des IgG anti-VCA, dans le sérum humain, renforce la valeur diagnostic (particulièrement intéressante), pour établir une primo-infection par EBV.

Selon l'isotype des Ig anti-VCA, détecté dans le sérum à analyser, il est possible de distinguer les porteurs sains du virus (prévalence des IgG), les primo-infections (prévalence des IgM) ou l'émergence d'une tumeur, notamment carcinome du nasopharynx (prévalence des IgA).

De manière surprenante, le réactif selon l'invention permet effectivement de détecter les IgA anti-VCA.

La présente invention a également pour objet un procédé de détection précoce du cancer du nasopharynx par une méthode immuno-enzymatique, caractérisé en ce qu'il comprend:
- la fixation d'un réactif selon l'invention sur un support, tel qu'une microplaque de titration,
- l'addition du sérum à analyser,
- la détection de la fixation des anticorps anti-VCA, éventuellement présents dans ledit sérum, par addition d'anticorps anti-IgA humaines couplés à une enzyme, et
- la révélation qualitative ou quantitative au spectrophotomètre par addition du substrat de l'enzyme.

La présente invention a, en outre, pour objet un kit ou coffret de diagnostic d'une infection virale, caractérisé en ce qu'il comprend au moins un réactif de diagnostic selon l'invention.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

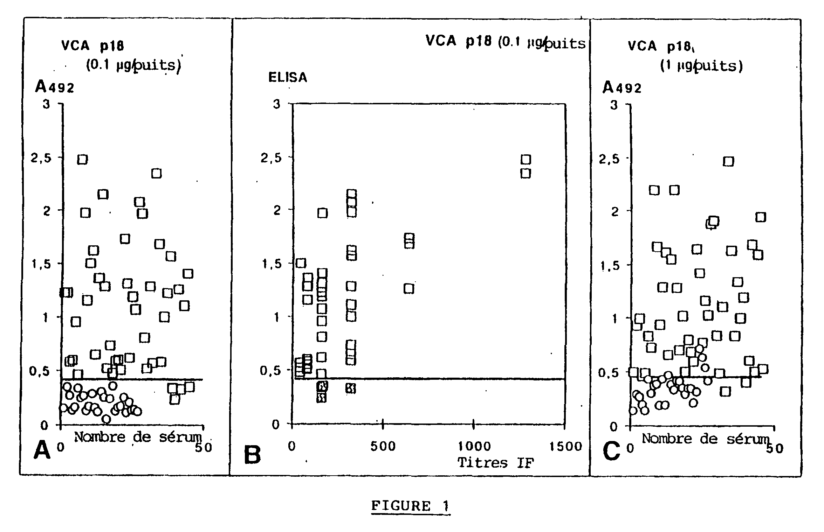

- la figure 1 illustre la réactivité des Ig-G-A-M de 46 sérums EBV-positifs (-□-) et de 28 sérums EBV-négatifs (-○-) caractérisés par immunofluorescence avec le peptide SEQ ID n°2 fixé sur un support solide à raison de 0,1 µg/puits (figures 1A et 1B) ou à raison de 1 µg/puits (figure 1C). La ligne horizontale représente la valeur seuil, correspondant à la moyenne obtenue avec les sérums négatifs (EBV-négatifs) + 3 déviations standards (SD). La figure 1B illustre plus particulièrement la comparaison entre les titres obtenus en IgG anti-VCA par un test d'immunofluorescence (IF) avec la valeur d'absorbance obtenue (A492) avec un test ELISA mettant en oeuvre le peptide SEQ ID n°2. A la figure 1B, le symbole -■- représente les sérums EBV-positifs SEQ ID n°2-négatifs. Les figures 1A et 1C comportent en abscisse le nombre de sérums et en ordonnée l'absorbance à 492 nm, alors que la figure 1B comporte en abscisse les titres IF et en ordonnée les absorbances obtenues en ELISA.

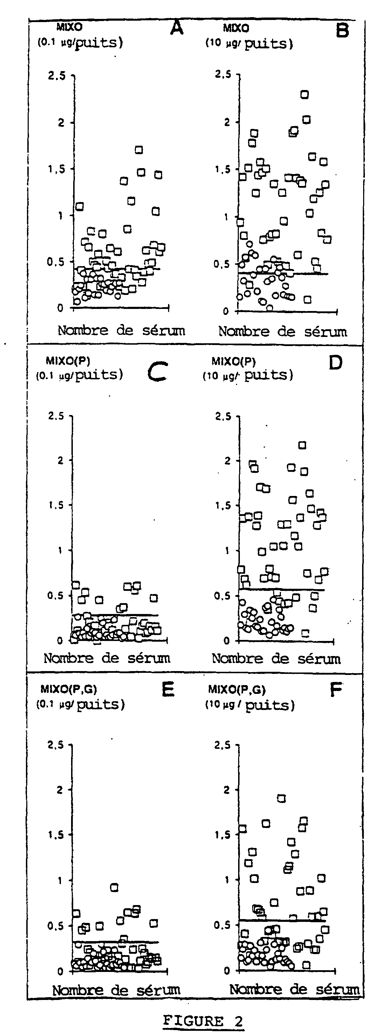

- la figure 2 représente l'effet des mixotopes MIXO, MIXO(P) et MIXO(P,G) sur la réactivité des IgG-A-M des sérums EBV-positifs (-□-) et les sérums EBV-négatifs (-○-) à deux concentrations différentes : 0,1 µg/puits (figures 2A, 2C et 2E) ou à 10 µg/puits (figures 2B, 2D, 2F), dans un test ELISA. La ligne horizontale représente la valeur seuil correspondant à la moyenne des sérums contrôles + 3 SD. Ces différentes figures comprennent en abscisse le nombre de sérums et en ordonnée l'absorbance à 492 nm.

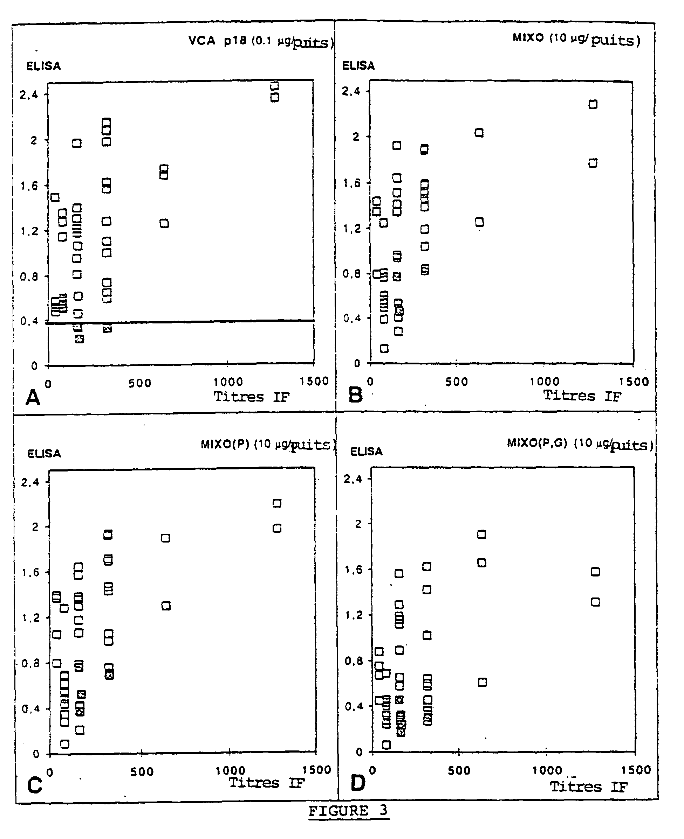

- la figure 3 illustre la comparaison des comportements de sérums EBV-positifs vis-à-vis du peptide VCAp18/SEQ ID n°2 (figure 3A) et des différents mixotopes : MIXO (figure 3B), MIXO(P) (figure 3C), MIXO(P,G) (figure 3B); les faux-négatifs obtenus avec le peptide VCAp18/SEQ ID n°2 (figure 3A, -■-) définissent la valeur seuil. Les données de ces figures 3 représentent les valeurs d'absorbance obtenues par ELISA de tous les sérums EBV-positifs, en fonction de leur titre IFA. Le peptide VCAp18/SEQ ID n°2 et les mixotopes sont utilisés en phase solide respectivement aux concentrations de 0,1 et 10 µg/puits.

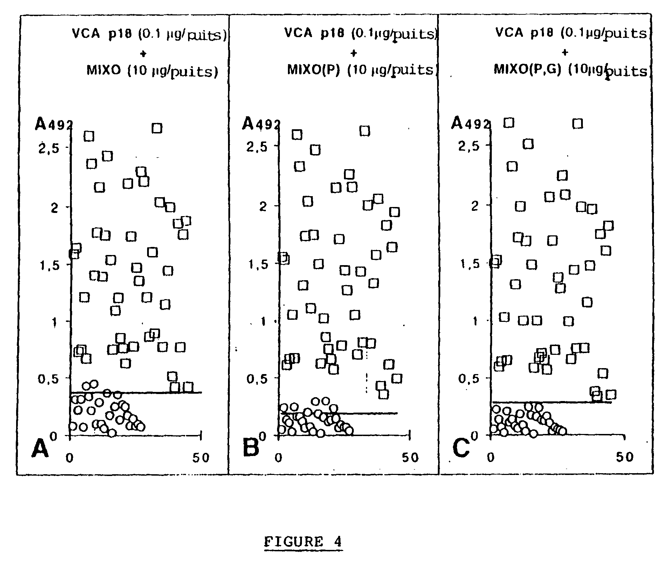

- la figure 4 illustre l'effet de l'association peptide VCAp18/SEQ ID n°2 + mixotopes : MIXO (figure 4A), MIXO(P) (figure 4B) ou MIXO(P,G) (figure 4C), sur la réactivité IgG-A-M des sérums EBV-positifs (-□-) et EBV-négatifs (-○-) dans des tests ELISA. Chaque puits de plaque de microtitration est séquentiellement revêtu de 0,1 µg de peptide VCAp18/SEQ ID n°2 et de 10 µg de l'un des mixotopes précités et mis en contact avec les sérums.

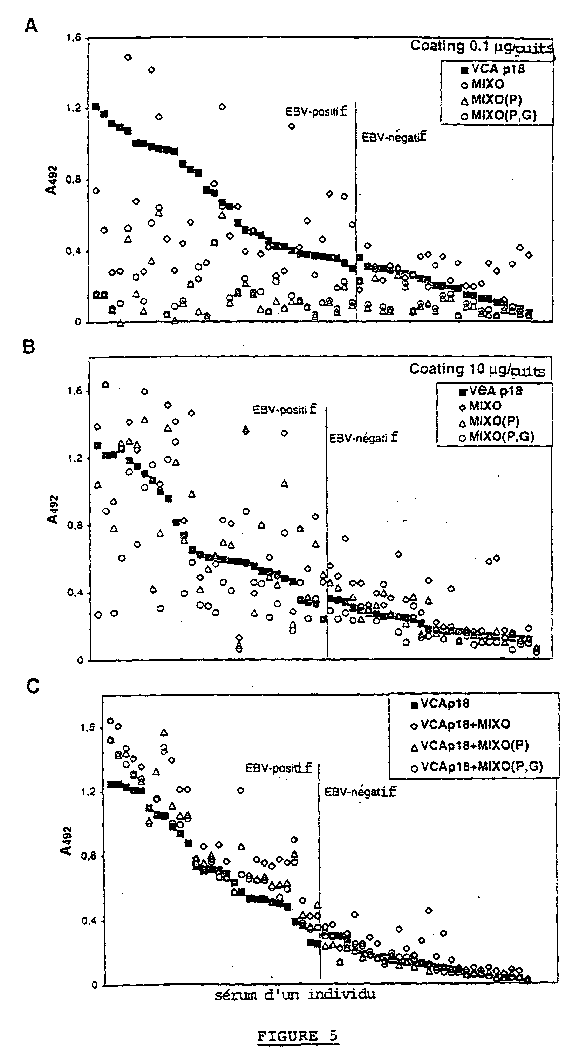

- les figures 5A et 5B illustrent l'évolution des réponses sérologiques humaines du peptide VCAp18/SEQ ID n°2 (-■-) et des trois mixotopes précités (MIXO (- 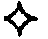 -) MIXO(P) (-Δ-) et MIXO(P,G) (-○-) à deux concentrations de revêtement différentes ; la figure 5C illustre l'évolution des réponses sérologiques du peptide VCAp18/SEQ ID n°2 (-■-) et des trois mixotopes précités en combinaison avec le peptide VCAp18/SEQ ID n°2: VCAp18/SEQ ID n°2+MIXO (- 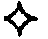 -), VCAp18/SEQ ID n°2+MIXO(P) (-Δ-) et VCAp18/SEQ ID n°2+MIXO(P,G) (-○-). Pour chaque expérience les sérums présentant une absorbance à 492 nm inférieure à 1,3 avec le peptide VCAp18/SEQ ID n°2 sont sélectionnés et la ligne verticale délimite la frontière entre les sérums EBV-positifs et EBV-négatifs.

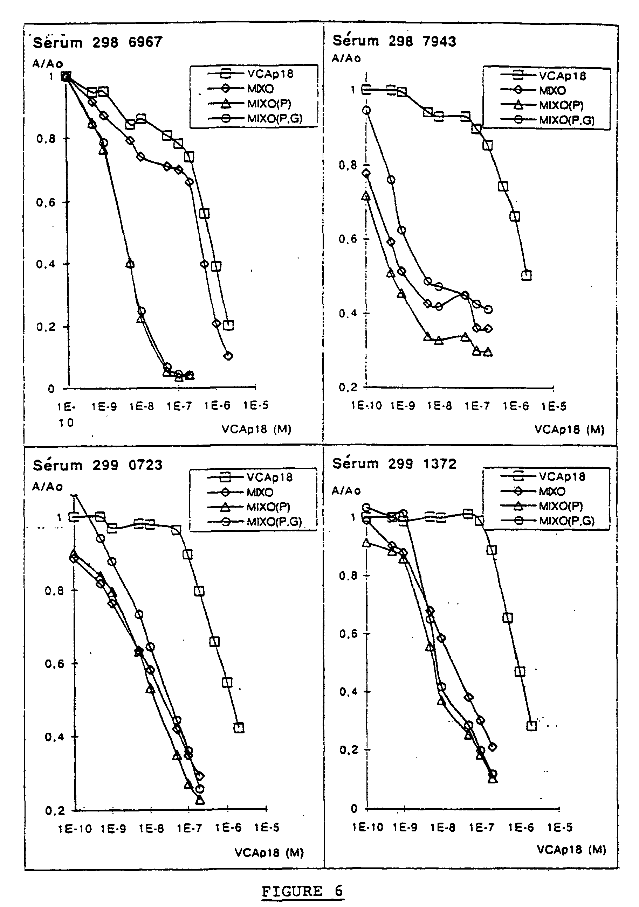

- la figure 6 illustre les inhibitions de liaison des anticorps de différents sérums EBV-positifs (sérum 298 6967, sérum 298 7943, sérum 299 0723 et sérum 299 1372) à une phase solide contenant le peptide VCAp18/SEQ ID n°2 (-□-), MIXO (- 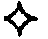 -), MIXO(P) (-Δ-) ou MIXO(P,G) (-○-) en augmentant les concentrations de peptide VCAp18/SEQ ID n°2. Cette figure comprend en abscisse la concentration en peptide VCAp18/SEQ ID n°2 et en ordonnée le rapport A/Ao.

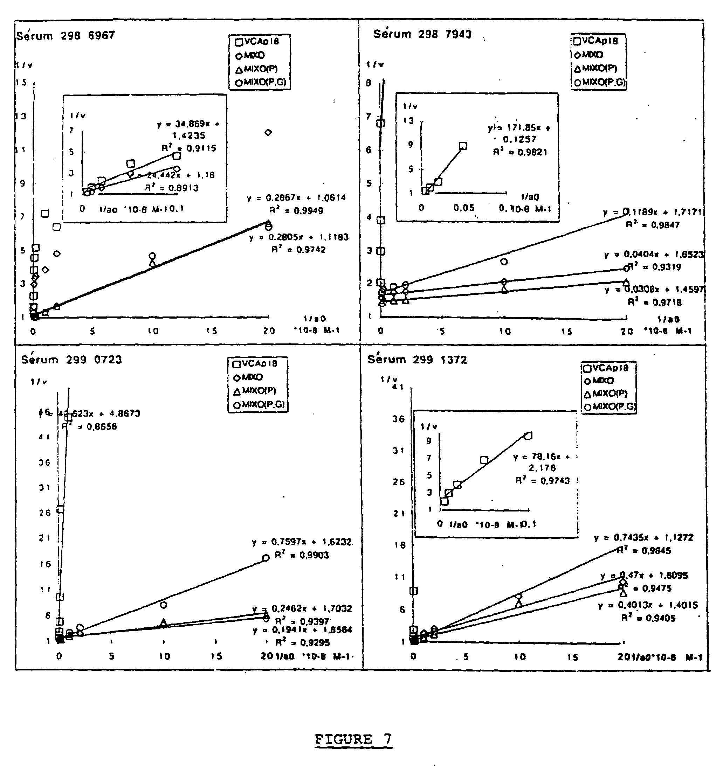

- la figure 7 représente des plots Klotz illustrant la liaison de l'anticorps de différents sérums EBV-positifs (sérum 298 6967, sérum 298 7943, sérum 299 0723 et sérum 299 1372) à des phases solides contenant le peptide VCAp18/SEQ ID n°2 (-□-), MIXO (- 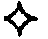 -), MIXO(P) (-Δ-) ou MIXO(P,G) (-○-).

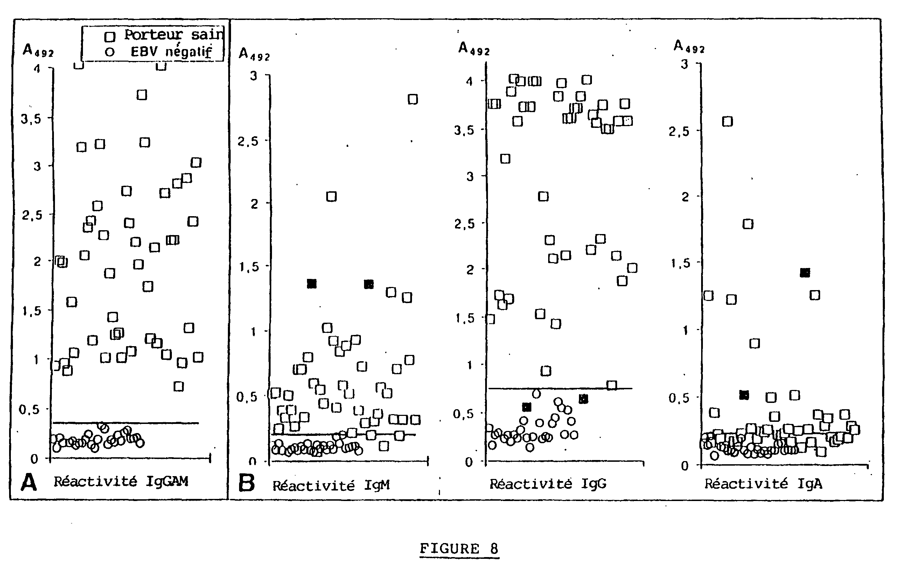

- la figure 8 illustre la réactivité IgG-A-M (figure 8A) et les réactivités séparées des différents isotypes G, A et M (figure 8B) de sérums EBV-positifs (sujets ayant eu une infection dans le passé, -□-) et de sérums EBV-négatifs (-○-), vis-à-vis du réactif selon l'invention VCAp18/SEQ ID n°2 + MIXO(P,G).

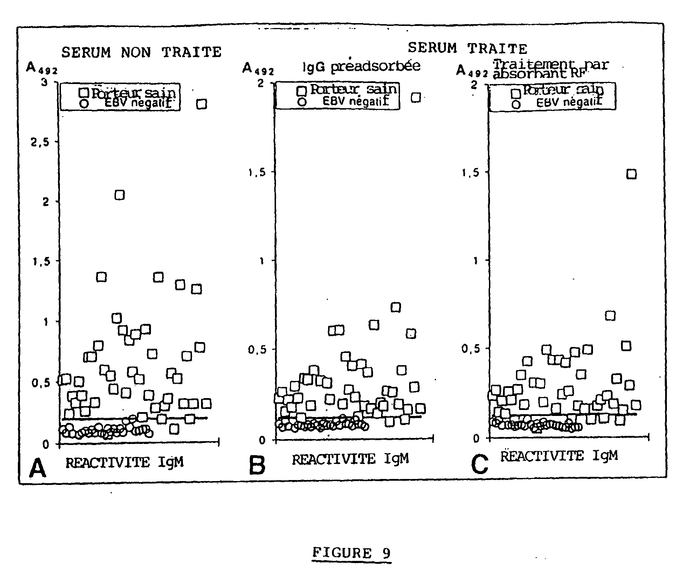

- la figure 9 illustre les réactivités de l'isotype IgM vis-à-vis du réactif selon l'invention VCAp187SEQ ID n°2 + MIXO(P,G) dé sérums EBV-positifs (infection dans le passé, -**□**-) et de sérums négatifs (-○-), non-traités (figure 9A), traités par des anticorps anti-IgG (figure 9B) ou traités par pré-adsorption des IgG (figure 9C).

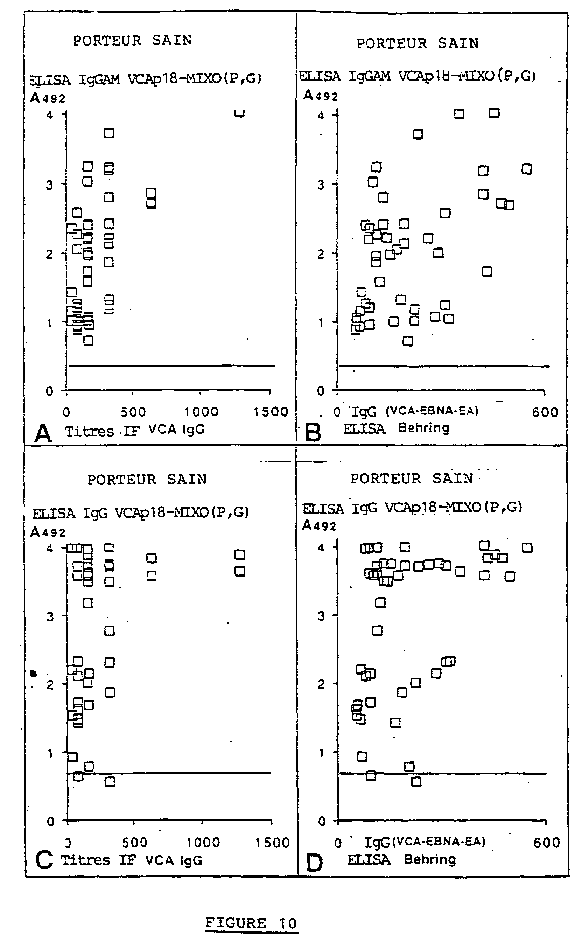

- la figure 10 illustre la comparaison des réactivités IgG-A-M (figures 10A et 10B) et IgG (figures 10C et 10D), de sérums humains de patients ayant eu une infection dans le passé, vis-à-vis du réactif selon l'invention VCAp18/SEQ ID n°2 + MIXO(P,G) avec un test IFA pour la détection des IgG-anti-VCA et un test ELISA Behring pour la détection des IgG-anti-(VCA-EBNA-EA). Les données représentent les valeurs d'absorbance obtenues en ELISA pour tous les sérums EBV-positifs, en fonction de leur titre IFA ou des valeurs d'absorbance obtenues avec le test ELISA Behring.

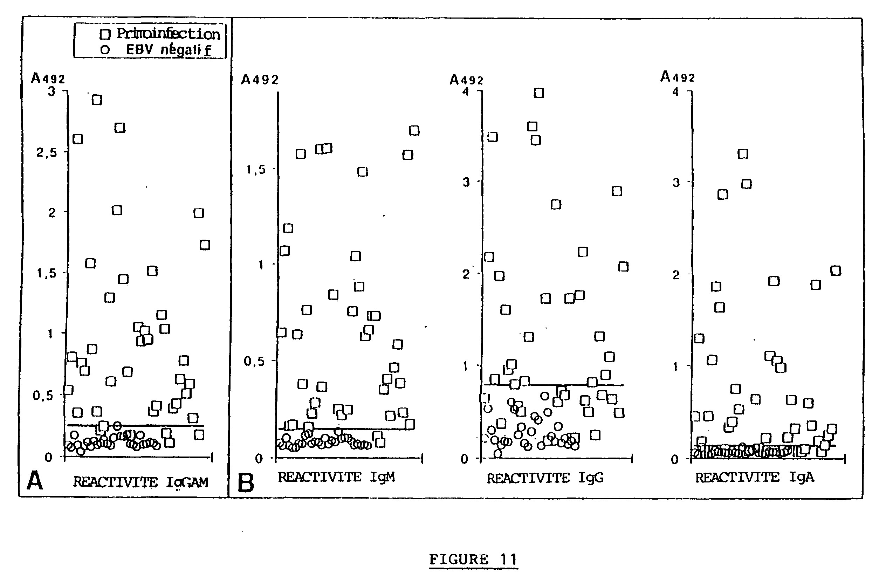

- la figure 11 illustre la réactivité IgG-A-M (figure 11A) et les réactivités séparées des différents isotypes G, A et M (figure 11B) de sérums EBV-positifs (primo-infection, -□-) et de sérums EBV-négatifs (-○-), vis-à-vis du réactif selon l'invention VCAp18/SEQ ID n°2 + MIXO(P,G).

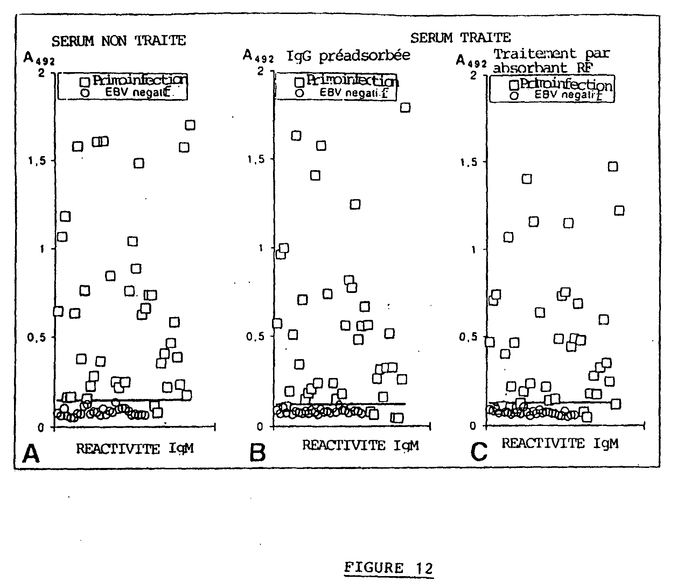

- la figure 12 illustre les réactivités de l'isotype IgM vis-à-vis du réactif selon l'invention VCAp18/SEQ ID n°2 + MIXO(P,G) de sérums EBV-positifs (primo-infection, -□-) et de sérums EBV-négatifs (-O-) non-traités (figure 12A), traités par des anticorps anti-IgG (figure 12B) ou traités par pré-adsorption des IgG (figure 12C).

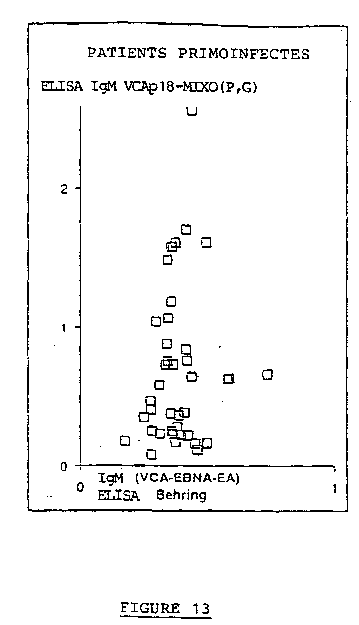

- la figure 13 illustre la comparaison de la réactivité IgM de sérums de patients primo-infectés vis-à-vis du réactif selon l'invention VCAp18/SEQ ID n°2 + MIXO(P,G) avec un test ELISA Behring pour la détection des IgG-anti-(VCA-EBNA-EA).

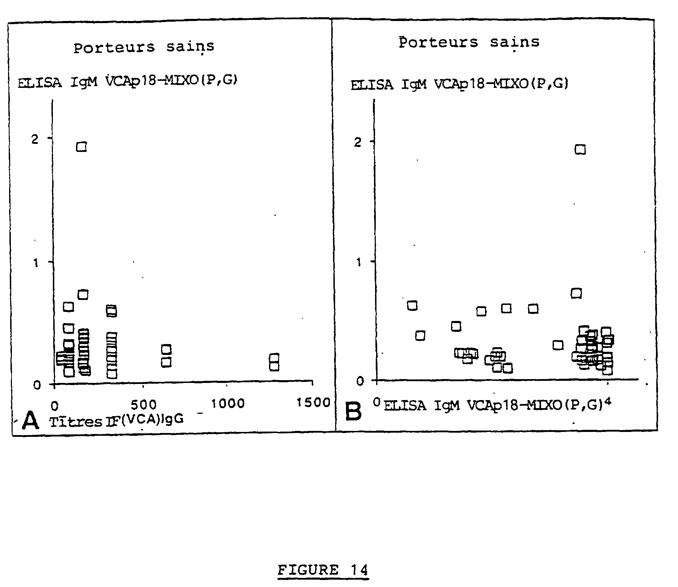

- la figure 14 illustre la comparaison de la détection des IgG dans des sérums de sujets ayant été infectés dans le passé, obtenue par IFA (figure 14A) ou par un test ELISA mettant en oeuvre le réactif selon l'invention VCAp18/SEQ ID n°2 + MIXO(P,G) (figure 14B) avec la réactivité IgM de sérums de patients ayant eu une infection dans le passé, obtenue avec un test ELISA utilisant un réactif selon l'invention VCAp18/SEQ ID n°2 + MIXO(P,G).

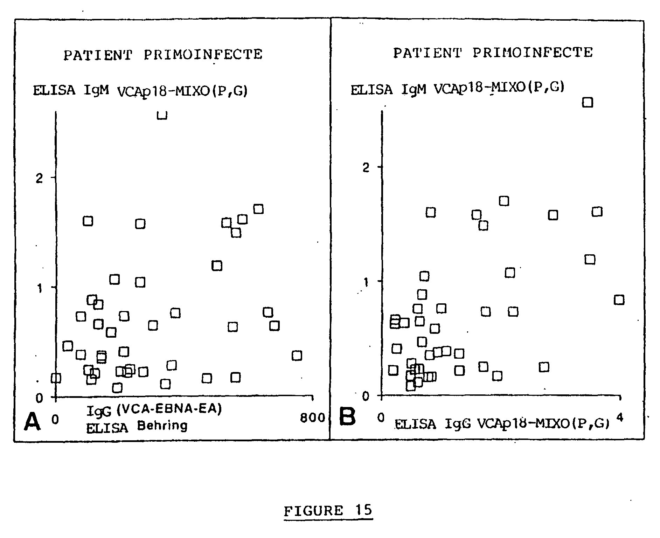

- la figure 15 illustre la comparaison de la valeur d'absorbance obtenue pour la détection des IgG dans des sérums de sujets primo-infectés, avec un test ELISA Behring VCA-EBNA-EA ou un test ELISA VCAp18/SEQ ID n°2 + MIXO(P,G) selon l'invention avec la réactivité IgM obtenue à l'aide d'un test ELISA utilisant un réactif selon l'invention VCAp18/SEQ ID n°2 + MIXO(P,G).
- la figure 16 illustre une matrice de remplacement des aminoacides, modifiée par rapport à celle de H.M. GEYSEN (référence précitée) et tient compte de la symétrie ; les valeurs en caractères gras sont présentes dans la matrice de Geysen, les valeurs 0 non significatives statistiquement sont maintenues arbitrairement ou remplacées par la valeur obtenue par le remplacement symétrique.

Il doit être bien entendu, toutefois, que ces exemples sont donnés uniquement à titre d'illustration de l'objet de l'invention, dont ils ne constituent en aucune manière une limitation.

### EXEMPLE 1 : Préparation des réactifs selon l'invention.

### a) Synthèse du peptide :

Le peptide VCAp18/SEQ ID n°2 est synthétisé en utilisant la stratégie en phase solide conventionnelle du type Boc-benzyle (ou Fmoc), dans un synthétiseur de peptides automatisé (modèle 430A, Applied Biosystems Inc.). Les groupes de protection des chaînes latérales sont les suivants : Asn (Trt), Gln (Trt), Asp (OChx), Glu (OChx), Ser (Bzl), Thr (Bzl), Arg (Tos), Cys (4-MeBzl), et His (Dnp) (voir R.C. SHEPPARD, Peptide, Synthesis, Comp. Org. Chem., 1979, 5, 321-363).

Les aminoacides sont introduits en utilisant le protocole d'activation HBTU/HOBt avec un double couplage systématique sur une résine Boc-Gln-Pam (Applied Biosystems). Après thiolyse du groupe dinitrophényl Dnp, suivie par une déprotection finale et un clivage à l'acide fluorhydrique, le peptide clivé et déprotégé est précipité et lavé avec du diéthyléther froid, puis dissous dans de l'acide acétique à 5 % et lyophilisé.

Le peptide est purifié à plus de 90 % sur une colonne de 5 mm x 250 mm, 100 A Nucleosil C18 RP-HPLC préparative (Macherey Nagel, Düren, FRG) et ledit peptide est ensuite caractérisé.

L'homogénéité est confirmée par HPLC analytique sur une colonne Vydac C18 éluée avec un système de solvants (TFA-acétonitrile-eau), dans un appareil Shimadzu. L'identité est confirmée par la détermination de la composition en amino-acides et par spectrométrie de masse enregistrée sur un spectromètre de masse Bio Ion 20 plasma (Bio Ion AB, Upsala, Suède) ([M+H]⁺ calc. : 2494.7 ; trouvée : 2495.4).

### b) Préparation des différents mixotopes :

Ceux-ci sont préparés comme décrits dans H. GRAS-MASSE et al., Peptide Research, 1992, 5, 4, 211-216.

Brièvement, des quantités équimolaires d'acides aminés protégés sont pesées et sont utilisées dans les réactions de couplage.

Pour compenser les différences de cinétique dans les réactivités des différents amino-acides, un premier couplage est réalisé avec 1 mmol (quantité totale) de Boc-amino-acide (ou d'un mélange de Boc-amino-acides). Un deuxième couplage, utilisant 2 mmol (quantité totale), est alors systématiquement réalisé. Après un clivage à l'acide fluorhydrique, le peptide brut est dissous dans du TFA (30 ml) et précipité par ajout dudit peptide dans une solution de diéthyléther froid (300 ml).

Après centrifugation, le précipité est dissous dans l'eau et lyophilisé. Après oxydation à l'air de la solution à pH neutre, les mixotopes sont purifiés par filtration sur gel sur une colonne TSK HW40S (Merck, Darmstadt, FRG). Un aliquot de chaque mixotope purifié est soumis à une hydrolyse acide totale pendant 24 heures avec un mélange HCl 6N:phénol (10:1), pour la détermination de la composition en amino-acides.

### c) Exemples de différents réactifs conformes à l'invention:

La séquence du peptide SEQ ID n°2 et des mixotopes qui en dérivent sont représentés au Tableau I ci-après.

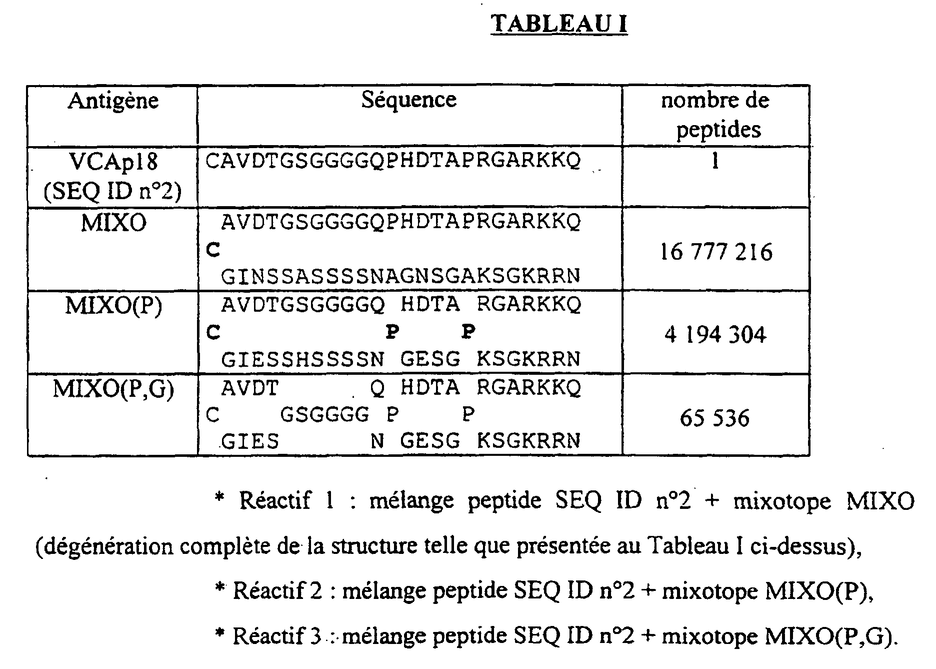

Ces réactifs sont, de préférence, fixés sur support solide (microplaque) à une concentration de 0,1 µg/puits, pour le peptide VCAp 18/SEQ ID n°2 et à une concentration de 10 µg/puits pour les mixotopes.

### EXEMPLE 2 : Test immuno-enzymatique mettant en oeuvre un réactif selon l'invention pour la sérodétection de l'EBV.

### A. Matériel et méthodes :

### - ELISA :

Des puits de plaques de microtitration (Nunc, Maxisorp, Rocksilde, Danemark) sont recouverts pendant une nuit à 4°C, soit avec 0,2 ml de peptide VCAp18/SEQ ID n°2, soit avec un mixotope (0,5 µg/ml dans 50 mM de NAHCO₃, pH 9,6), soit séquentiellement avec 0,2 ml de peptide VCAp18/SEQ ID n°2 (0,5 µg/ml) et 0,2 ml de mixotope (50 µg/ml).

Chaque puits est ensuite lavé avec un tampon phosphate 0,01 M comprenant du NaCl 1,8 %, pH 7,4 (PBS) et les sites de liaison en excès sont bloqués par de l'albumine (addition de 0,3 ml de BSA 2 % (sérum albumine bovine) dans du PBS, à 37°C, pendant 60 minutes).

Après 3 lavages avec 0,3 ml de PBS 0,5 %-Tween 20 (Sigma) (tampon PBS-T), les sérums humains à tester sont dilués au 1/50 dans du PBS-T comprenant de la sérum albumine bovine (BSA) 2 % et sont incubés dans des puits contenant le réactif selon l'invention comme précisé ci-dessus, pendant 120 minutes à 37°C, dans une atmosphère humidifiée.

Après 4 lavages, les conjugués peroxydase-anticorps de chèvre-anti-IgG-A-M humaines (Diagnostic Pasteur), dilués au 1/10 000 dans du tampon PBS-T comprenant de la BSA 2 %, sont incubés pendant 60 minutes à 37°C.

L'anticorps conjugué, qui se lie aux Ig fixées sur le support, est révélé pour son activité peroxydase, en utilisant comme substrat du dihydrochlorure d'*o*-phénylènediamine et de l'H₂O₂, dans un tampon citrate 0,05 M, pH 5,5, pendant 30 minutes à l'obscurité et à température ambiante.

La réaction est bloquée par l'addition d'H₂SO₄ 4N (50 µl). L'absorbance est enregistrée contre un blanc à 492 nm A₄₉₂), avec un lecteur automatique multicanaux (M_{R} 5000, Dynatech).

La moyenne A₄₉₂ + 3 déviations standards (SD) des échantillons EBV- négatifs est utilisée comme valeur seuil dans les tests ELISA.

### - Mesure de l'avidité de la liaison à l'anticorps :

La spécificité de liaison des sérums positifs aux différents mixotopes en phase solide est évaluée par l'absorption des anticorps par l'antigène VCAp18/SEQ ID n°2 natif en solution, en utilisant la méthode de B. FRIGUET et al. (J. Immunol. Methods, 1985, **77**, 305-319).

Cette méthode est basée sur la mesure de la concentration en anticorps libre par une méthode ELISA indirecte, lorsque l'antigène VCAp18/SEQ ID n°2 et les anticorps sont à l'équilibre en solution.

L'antigène VCAp18/SEQ ID n°2, à différentes concentrations (10⁻¹⁰ M à 2.10⁻⁶ M) est d'abord incubé en solution (tampon PBS-T + BSA 2 %) avec un sérum EBV-positif, à concentration constante (1/50) jusqu'à l'atteinte de l'état d'équilibre.

Après une incubation pendant 18 heures à 4°C, 200 µl de chaque mélange est transféré et incubé pendant 60 min à 20°C dans les puits d'une plaque de microtitration, préalablement recouverte de peptide VCAp18/SEQ ID n°2 (0,2 ml, correspondant à 0,5 µg/ml) ou d'un mixotope (50 µg/ml, 0,2 ml), dans du NAHCO₃ 50 mM, pH 9,6.

Après lavage avec du tampon PBS-T, les immunoglobulines liées sont détectées par addition d'anticorps de chèvre anti-IgG-A-M humaines, couplés à de la peroxydase.

L'anticorps conjugué, qui se lie aux Ig, est révélé par l'activité peroxydase comme décrit ci-dessus. Cette méthode donne les courbes de déplacement de la liaison A/Ao par rapport à log(ao). Une estimation précise de l'avidité moyenne du sérum contenant les anticorps anti-VCAp18 est donnée par l'équation Ao/(Ao-A)=1/ν=1+*Kd*/ao, dans laquelle ao est la concentration en antigène soluble total, A et Ao sont les absorbances à 492 nm avec ou sans antigène bloquant, respectivement et ν est la fraction d'anticorps lié, si les différentes conditions exposées dans FRIGUET et al. sont satisfaites.

### B. Résultats

### a) Liaison anticorps sériques-domaine C-terminal du peptide VCAp18/SEQ ID n°2 (ELISA VCAp18/SEQ ID n°2) :

La réactivité des Ig G-M-A humaines anti-VCA vis-à-vis du peptide VCAp18/171-194 est analysée par un test ELISA, sur différents sérums: 46 sérums EBV-positifs et 28 sérums EBV-négatifs, sélectionnés après réalisation d'un test ELISA Behring et confirmés par IFA (détection des IgG anti-VCA, Immunoconcept, USA).

Comme précisé dans W.M.J. Van GRUNSVEN, J. Infect. Dis., 1994, **170**, 13-19, le domaine C-terminal de la protéine VCAp18 montre une importante immunoréactivité (figure 1A).

Lorsqu'il recouvre, à une concentration de 0,1 µg/puits, des plaques de microtitration, ce peptide VCAp18/SEQ ID n°2 est reconnu par les anticorps de la plupart des sérums EBV-positifs.

En utilisant 3 déviations standards au-dessus de la valeur moyenne, obtenue avec les sérums négatifs contrôles, comme limite inférieure de détection, une sensibilité de liaison élevée (89 %) et une spécificité optimale (100 %) sont observées dans l'analyse de la réponse anti-VCAp18 (figure 1A).

Pour évaluer la possibilité d'augmenter la sensibilité du test, en fonction de la quantité de peptides recouvrant les plaques, une autre concentration a été testée pour le *coating* (quantité recouvrant les plaques), à savoir 1 µg/puits ; dans un tel cas, on observe une diminution de la spécificité à 89 % (figure 1C).

La comparaison des titres obtenus en IF et des valeurs d'absorbance obtenues en ELISA, pour chaque sérum, ne montre pas de corrélation claire, essentiellement en raison d'une variabilité importante des absorbances obtenues en ELISA pour les sérums présentant des titres IF intermédiaires ou faibles (figure 1B).

Les sérums EBV-positifs, qui échappent à la détection par ELISA (-■- dans la figure 1B), montrent des titres en IF détectables au 160^{ème} ou au 320^{ème} (dilutions limites), confirmant que l'antigène VCA est effectivement reconnu par ces quatre sérums peptide VCAp 18-négatif.

### b) Liaison anticorps sériques mixotopes (ELISA VCA mixotope) :

Les mixotopes ont été testés comme antigènes en phase solide, à deux concentrations , à savoir 0,1 µg et 10 µg/puits (figure 2).

Après revêtement des puits à une concentration de 0,1 µg, la sensibilité est clairement insuffisante : seulement 26, 10 et 10 des 46 sérums IgG-A-M-positifs réagissent avec MIXO, MIXO(P) et MIXO(P,G) respectivement (figures 2A, C,E).

Après revêtement avec les mixotopes à une concentration de 10 µg/puits, la détection des sérums positifs est beaucoup plus sensible avec une augmentation des absorbances. 46, 36 et 27 des 46 sérums EBV-positifs sont détectés avec les mixotopes MIXO, MIXO(P) et MIXO(P,G) respectivement (figures 2B, D, F). Cependant, la valeur seuil est également plus élevée, en raison d'une variation accrue des absorbances observées avec les sérums contrôles négatifs.

De manière surprenante, la perte de la spécificité de liaison (11 sérums faussement positifs), est observée seulement lorsqu'on utilise l'antigène MIXO (figures 2B, D, F), aucun faux-positif n'est observé avec les deux autres mixotopes.

La comparaison des titres IF et des valeurs d'absorbance obtenues en ELISA pour chacun des 46 sérums IFA-positifs sont illustrés à la figure 3. Les absorbances observées en ELISA sont augmentées par rapport aux résultats obtenus avec le test IF ; ces résultats sont particulièrement intéressants pour les sérums présentant des titres en IFA faibles ou intermédiaires.

Les tests VCAp18-négatifs IF-positifs sont symbolisés par des carrés noirs, à la figure 3.

### c) Liaison anticorps sériques-réactif selon l'invention (combinaisons peptide VCAp18/SEQ ID n°2 + mixotope) (ELISA VCAp18/SEQ ID n°2 + mixotope) :

Pour montrer l'augmentation de la sensibilité de la détection à l'aide du réactif selon l'invention, on utilise des combinaisons comprenant le peptide VCAp18/SEQ ID n°2 recouvrant une plaque de microtitration à raison de 0,1 µg/puits avec différents mixotopes recouvrant la plaque de microtitration à raison de 10 µg/puits.

Comme illustré à la figure 4 (A, B, C), tous les sérums EBV-positifs sont détectés avec les trois combinaisons.

Une hétérogénéité des résultats, avec le test ELISA est observée avec les sérums EBV-négatifs, lorsque la dégénération du mixotope augmente. Comme illustré aux figures 4A et 4B, les combinaisons MIXO et MIXO(P) avec le peptide VCAp18/SEQ ID n°2 entraînent la présence de 4 ou 5 sérums faussement positifs. Néanmoins, la combinaison avec l'antigène MIXO(P,G) fournit une sensibilité de liaison optimale (100 %) et une spécificité optimale (100 %) dans le séro-diagnostic immunoenzymatique VCA-EBV (figure 4C).

Les résultats obtenus sont illustrés au Tableau II ci-après.

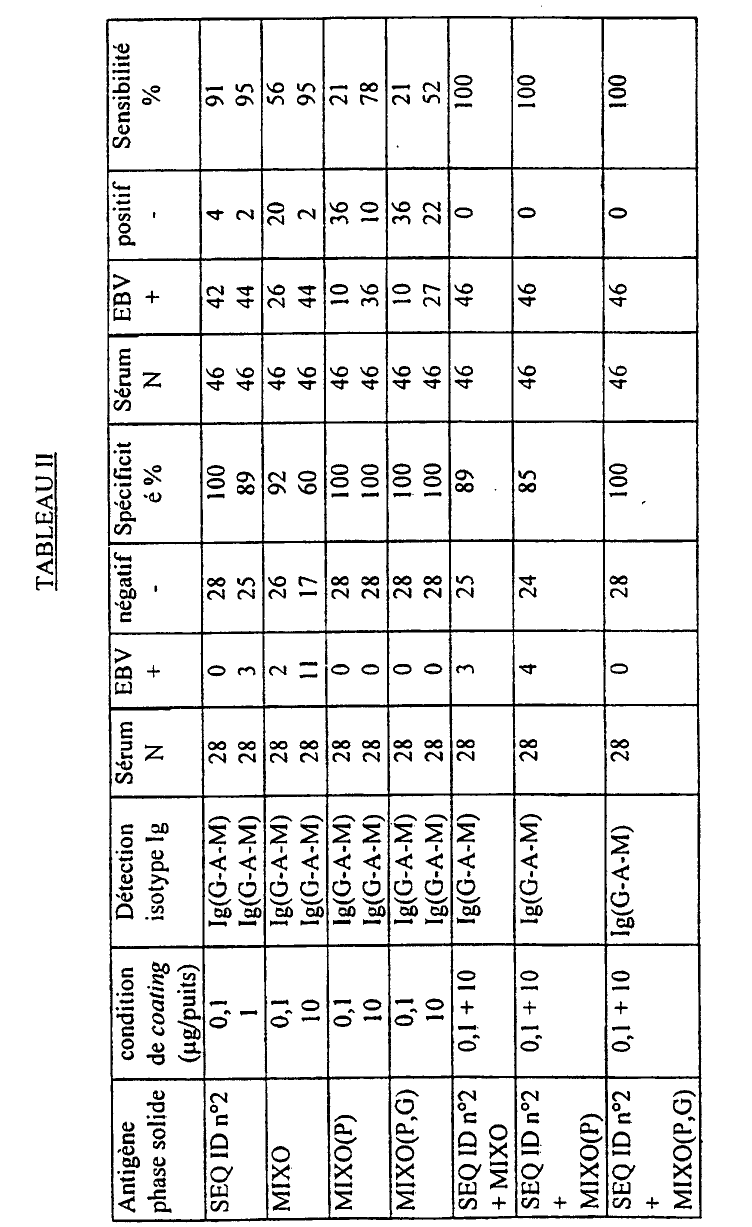

### d) Pertinence des combinaisons peptide VCAp18/mixotopes pour le sérodiagnostic de l'EBV:

Une analyse plus détaillée de la distribution des réponses individuelles au peptide VCAp18/SEQ ID n°2, à ses mixotopes et à leurs combinaisons (réactif selon l'invention) est illustrée à la figure 5.

Tous les sérums présentant une valeur d'absorbance inférieure à 1,3 avec un test ELISA ne mettant en oeuvre que le peptide VCAp18/SEQ ID n°2 ont été sélectionnés. Chaque sérum est représenté par 4 symboles correspondant aux absorbances obtenues lorsqu'on les fait réagir avec les différents antigènes en phase solide.

Dans certains cas, le signal est clairement augmenté, lorsqu'on utilise les mixotopes comme antigènes en phase solide y compris pour les sérums IF-positifs-VCAp18/SEQ ID n°2 négatifs.

Comme illustré à la figure 5C, presque toutes les absorbances obtenues, lorsque l'on teste les sérums positifs avec le réactif selon l'invention, sont supérieures aux absorbances obtenues avec le peptide VCAp18/SEQ ID n°2 seul.

Les 3 combinaisons détectent de manière efficace tous les sérums EBV-positifs, qui présentent un signal faible avec les mixotopes.

Le signal obtenu avec les sérums négatifs avec les combinaisons selon l'invention est également généralement plus faible que lorsque ces sérums sont testés avec le peptide VCAp18/SEQ ID n°2 seulement, excepté lorsque l'on utilise la construction totalement dégénérée MIXO.

Toutefois, les mixotopes complètement dégénérés, obtenus à partir de peptides issus de la partie C-terminale de la protéine VCAp18 et ne contenant pas de proline, par exemple, confèrent effectivement les propriétés recherchées, au réactif selon la présente invention.

### e) Évaluation de l'avidité des mixotopes pour les anticorps sériques comparée au peptide VCAp18/SEQ ID n°2, par ELISA :

Le peptide VCAp18/SEQ ID n°2 est utilisé dans des expériences d'inhibition ELISA pour évaluer sa capacité à inhiber la liaison des sérums humains EBV positifs aux différents mixotopes, utilisés en phase solide.

La figure 6 représente quelques essais d'inhibition.

Comme illustré à cette figure 6, la liaison des sérums humains aux 3 mixotopes peut être spécifiquement et fortement inhibée, en augmentant les concentrations en peptide VCAp18/SEQ ID n°2.

En outre, le peptide VCAp18/SEQ ID n°2 en phase solide et les différents mixotopes en phase solide, donnent des courbes de déplacement parallèle, indiquant la présence d'une population d'anticorps commune, sensible au peptide natif et aux peptides dégénérés.

La figure 7 représente les plots Klotz de liaison aux anticorps des sérums EBV-positifs, mesurée en ELISA par la méthode de FRIGUET et al. précitée. Les valeurs des constantes de dissociation sont déduites à partir de la régression linéaire des résultats, exprimés à la figure 6. On observe une augmentation d'un facteur de 10 à 400 des constantes d'avidité pour les différents mixotopes, comparés au peptide VCAp18/SEQ ID n°2.

La valeur moyenne de Kd pour VCAp18/171-194, MIXO, MIXO(P) et MIXO(P,G) dans ces expériences est respectivement de 0,81±0,62 mM, 0,06±0,01 mM, 2,40±1,54 nM et 4,75±3,75 nM.

### EXEMPLE 3 : Test immuno-enzymatique mettant en oeuvre un réactif selon l'invention pour la sérodétection différentielle des IgG, IgM et IgA humaines anti-VCA et son rôle dans le diagnostic de différentes pathologies.

### a) Liaison anticorps sériques réactif VCAp18/SEQ ID n°2 + MIXO(P,G) selon l'invention chez les porteurs sains :

La réactivité des IgG-A-M, des IgG, des IgA et des IgM humaines anti-VCA vis-à-vis du réactif selon l'invention VCAp18/SEQ ID n°2 + MIXO(P,G), est analysée à l'aide d'un test ELISA, en utilisant les sérums caractérisés en IF comme EBV-négatif et EBV-positif (46 EBV-positifs et 28 EBV-négatifs).

La valeur seuil est définie comme trois variations standards au dessus de la valeur moyenne des sérums EBV négatifs, déterminés pour chaque isotype.

Avec ce critère, aucun sérum faussement positif n'est observé dans aucun ELISA.

Comme illustré à l'exemple 1, c) ci-dessus (voir également figure 4C), le réactif peptide VCAp18/SEQ ID n°2 + MIXO(P,G), utilisé comme antigène en phase solide, montre une sensibilité de liaison optimale de 100 % et une spécificité de 100 % dans les diagnostics sérologiques IgG-A-M d'EBV par méthode immunoenzymatique.

Le même ensemble de sérums positifs par IF a été testé pour sa réactivité IgM, IgG et IgA, avec un test ELISA utilisant comme réactif un mélange VCAp18/SEQ ID n°2 + MIXO(P,G).

Pour réaliser les tests isotype-spécifiques, on procède comme suit:

On procède comme précisé à l'exemple 1, pour la préparation des plaques de microtitration.

Toutefois, avant l'incubation des sérums à analyser, dans les conditions précisées à l'exemple 1, les sérums utilisés pour l'analyse des IgM peuvent être traités :
- pour éliminer les IgG (préadsorption des IgG ou prétraitement avec un sérum anti-IgG humaines (Diagnostic Pasteur, Marnes-La-Vallée, France)) ou
- pour resolubiliser les IgM, avec une solution absorbant le facteur rhumatoïde (Behringwerke AG, Marburg, Allemagne),
conformément aux instructions des fabricants.

Pour la révélation, on procède comme suit :

Après 4 lavages, les conjugués anticorps (aussi appelés seconds anticorps) de chèvre dirigés contre les Ig(G-A-M), les IgG, les IgA et les IgM humaines (Diagnostic Pasteur, Marnes-La-Coquette, France), dilués au 1/10 000 dans un tampon PBS-T + BSA 2 % sont incubés 60 min à 37°C.

Les seconds anticorps conjugués qui se lient aux Ig sériques fixées sur le support sont révélés comme précisé à l'exemple 1.

Comme illustré à la figure 8B, tous les sérums à l'exception de 2 réagissent positivement avec les IgG ; toutefois, ces deux sérums IgG-négatifs ont des réactivités IgA et IgM élevées, comme indiqué par les carrés noirs dans la figure 8B. Ces résultats sont en accord avec la sensibilité de liaison de 100 % et la spécificité obtenue dans le test IgG-A-M (figure 4C ou figure 8A) et avec le fait que les sérums de porteurs sains (infection intervenue dans le passé), présentent une faible réactivité IgA et IgM (figure 8B).

Cependant, 91 % de ces sérums de sujets infectés dans le passé (porteurs sains) sont IgM-positifs, parmi lesquels 78 % ont des taux résiduels d'IgM (DO inférieure à 1), comparés à la valeur d'absorbance obtenue pour les IgG ; seulement 39 % desdits sérums sont IgA-négatifs ; ceci est un résultat surprenant dans la mesure où la plupart des patients étaient considérés comme des porteurs sains ou des convalescents, c'est-à-dire IgM et IgA-négatifs.

Seulement un sérum a été détecté comme ayant à la fois des taux élevés en IgM et en IgG, alors qu'il était IgA négatif, ceci indiquant probablement un patient réinfecté ou primo-infecté.

Pour montrer la spécificité de la réponse IgM, les sérums des porteurs sains ont été traités avec des réactifs de précipitation des IgG : sérum traité avec un sérum anti-IgG ou avec des réactifs de resolubilisation des IgM ou des réactifs permettant de décomplexer les IgM : sérum traité avec une solution apte à absorber le facteur rhumatoïde.

La figure 9 compare les trois ELISA réalisés (sérum non traité, sérum traité avec absorbant et sérum traité avec sérum anti-IgG). On ne détecte aucune différence significative, un signal plus faible étant observé pour l'ELISA effectué sur les sérums traités avec les réactifs précipitant les IgG.

Le test ELISA VCAp18/SEQ ID n°2 + MIXO(P,G) pour la détection des Ig(G-A-M) ou des IgG ont été comparés à la figure 10, à une méthode conventionnelle d'immunofluorescence mettant en oeuvre comme antigène le VCA ou une combinaison VCA-EBNA-EA pour la détection des IgG dans chacun des 46 sérums IFA positifs (méthode Behring).

Comme illustré aux figures 10A et 10C à des titres élevés IFA correspondent des valeurs d'absorbance élevées avec les tests ELISA VCAp18/SEQ ID n°2 + MIXO(P,G) pour la détection des Ig(G-A-M) ou des IgG.

Par ailleurs, une augmentation importante des valeurs obtenues avec le test ELISA VCAp18/SEQ ID n°2-MIXO(P,G) est observée pour les titres intermédiaires et faibles en IF.

Cette situation est particulièrement importante pour la détection des IgG-A-M et démontre la pertinence de la détection IgG-A-M (figures 10A, C).

Le même type de résultat est observé à la figure 10D, qui compare un test ELISA mettant en oeuvre le réactif VCAp18/SEQ ID n°2 + MIXO(P,G) et un ELISA commercial Behring, pour la détection des IgG.

Les deux sérums EBV-positifs, qui échappent à la détection à l'aide du réactif VCAp18/SEQ ID n°2-MIXO(P,G), présentent des titres IF faibles ou intermédiaires (80^{ème} et 320^{ème}) et sont en dessous de la valeur moyenne observée dans le test ELISA Behring pour la détection des IgG.

Ce résultat n'est pas inconsistant dans la mesure où un autre ensemble d'antigènes incluant EA et EBNA est utilisé dans le test Behring.

La figure 10B compare le test ELISA VCAp18/SEQ ID n°2 + MIXO(P,G), pour la détection des IgG-A-M selon l'invention et le test ELISA Behring, pour la détection des IgG. Aucune corrélation claire n'est observée entre les réactivités IgG et IgG-A-M, suggérant que la combinaison selon l'invention est très spécifique et contribue à la liaison de tous les isotypes humains, y compris le type M considéré comme l'isotype le moins affin et qui est produit en tout début de l'infection.

### b) Etude de la liaison des anticorps sériques au réactif selon l'invention VCAp18/SEQ ID n°2 + MIXO(P,G) chez les patients primo-infectés.

La réactivité des Ig G-A-M, des IgG, des IgA et des IgM humaines anti-VCA vis-à-vis du réactif selon l'invention VCAp18/SEQ ID n°2 + MIXO(P,G) est analysée à l'aide d'un test ELISA, en utilisant les 28 sérums EBV-négatifs sélectionnés comme contrôles et les 40 sérums EBV-positifs obtenus à partir de patients primo-infectés et donnant des résultats positifs avec un test ELISA (EBNA-VCA-EA) pour la détection des IgM et des résultats négatifs avec un test ELISA Behring pour la détection des IgG humaines anti-EBNA1.

Les résultats obtenus avec le test ELISA mettant en oeuvre le réactif selon l'invention VCAp18/SEQ ID n°2 + MIXO(P,G), pour la détection des IgG-A-M sont présentés à la figure 11A et une étude détaillée de la réponse isotypique des différents sérums est illustrée à la figure 11B.

Seulement six des sérums EBNA1-négatifs présentent un résultat au-dessus de la valeur seuil *(cut off)* pour ce qui concerne la réactivité vis-à-vis des IgG-A-M (figure 11A).

Tous ces sérums appartiennent au groupe constitué par les sérums IgG-négatifs (40 %). Deux d'entre eux ne réagissent avec aucun test et sont considérés comme des faux-négatifs.

De plus, ce sont les deux seuls à être IgM-négatifs (figure 11B). A la suite de cette IgM-négativité, ces deux sérums ont été testés en immunofluorescence (IF) pour confirmation de leur séropositivité. Les titres obtenus correspondent à des valeurs au 10ème et au 40ème. Ces titres, très faibles, indiquent une grande précocité de l'infection par EBV. Une explication pourrait être la détection d'un autre antigène tel que EA inclus dans les kits commercialisés ou plus probablement, une réaction croisée avec un autre virus du groupe Herpes (CMV, HSV...).

Par ailleurs, les 4 autres sérums qui sont également IgG-négatifs, présentent un taux d'IgM et d'IgA faible, indiquant une phase précoce de primo-infection.

La spécificité de la réactivité IgM a été étudiée, en neutralisant la réactivité IgG par des agents précipitants.

Comme illustré aux figures 12A et 12C, les sérums non-traités et les sérums traités (absorbant-RF) ne présentent pas de différence de réactivité.

Par contre, la préadsorption des IgG induit deux résultats faussement négatifs (figure 12B). Cependant, ces deux derniers sérums sont IgG-A-M-positifs. L'un d'entre eux s'est révélé seulement IgA-positif, alors que l'autre fournit une forte réponse IgG et une faible réponse IgA.

La figure 13 compare un test ELISA mettant en oeuvre un réactif selon l'invention (VCAp18/SEQ ID n°2 + MIXO(P,G) pour la détection des IgG et un test ELISA Behring pour la détection spécifique des IgM anti-EBV, dans lequel un extrait nucléaire des cellules infectées par un EBV est utilisé comme antigène.

On n'observe aucune corrélation entre les valeurs obtenues avec ces deux tests ELISA. Un déplacement des valeurs d'absorbance est observé avec le réactif selon l'invention. Un sérum présentant un résultat difficile à interpréter avec le test Behring est détecté de manière efficace avec les ELISA mettant en oeuvre un réactif selon l'invention, aussi bien pour la détection spécifique des IgM que des IgG anti-VCA.

### c) Pertinence du réactif selon l'invention VCAp18/SEQ ID n°2 + MIXO(P,G) pour le sérodiagnostic différentiel des isotypes produits lors d'une infection à EBV.

La discrimination entre une infection passée (porteur sain) et une primo-infection était jusqu'à présent basée sur la réactivité IgM et IgG obtenue avec les tests ELISA Behring mettant en oeuvre un réactif VCA+EBNA+EA, pour la détection des IgM et des IgG.

Les figures 14 et 15 illustrent les résultats comparés obtenus avec le réactif selon l'invention VCAp18/SEQ ID n°2 + MIXO(P,G) pour la détection de la réponse IgM humaine, dans deux populations EBV-positifs, étudiées en référence à leur taux d'IgG, déterminé par la méthode selon la présente invention et par les méthodes classiques.

Les valeurs d'absorbance obtenues avec des patients ayant présenté une infection dans le passé (porteurs sains) sont distribués selon l'axe des X (figure 14A). Cette situation est en accord avec les résultats obtenus en sérologie classique avec ce type de patients (IgM-négatifs et IgG-positifs, comme déterminé avec un test d'immunofluorescence).

Seulement l'un de ces sérums a donné un signal IgM-positif fort avec le réactif selon l'invention VCAp18/SEQ ID n°2 + MIXO(P,G), indiquant une primo-infection (figure 14A) ; en outre, on obtient un signal IgG important alors que par immunofluorescence, on n'observe qu'un faible signal IgG.

Ce résultat confirme une réinfection (ou une phase de convalescence), dans la mesure où la réponse IgA est négative ; ce résultat montre l'intérêt du réactif selon l'invention dans la détection des différents isotypes.

On observe, en outre, un déplacement vers la droite, le long de l'axe des X dans le test ELISA réalisé avec le réactif selon l'invention.

Dans le cas des patients primo-infectés, les valeurs obtenues avec les tests ELISA sont distribuées le long de l'axe des Y.

En fait, les figures 15 (A et B) montrent une répartition en diagonale.

Les isotypes M et G apparaissent séquentiellement. mais presque simultanément, après le début de l'infection.

En conséquence, il n'est pas surprenant de détecter l'isotype G dans les sérums de patients primo-infectés.

Un sous-ensemble, relativement important, de sérums, ne se situant ni dans la diagonale, ni le long de l'axe des Y, mais près de l'axe des X, indique une phase latente ou persistante de l'infection pour ces sérums, dans la mesure où la plupart d'entre eux sont IgA-positifs. Cette situation apparaît moins à la figure 15B dans laquelle on observe une baisse du nombre de sérums, le long de l'axe des X.

De manière surprenante, les réactifs selon l'invention permettent aussi bien la détection de la réactivité Ig globale (IgG-A-M) que la détection de la réactivité de chaque isotype (IgG, M ou IgA).

Les résultats décrits ci-dessus sont illustrés au Tableau III ci-après.

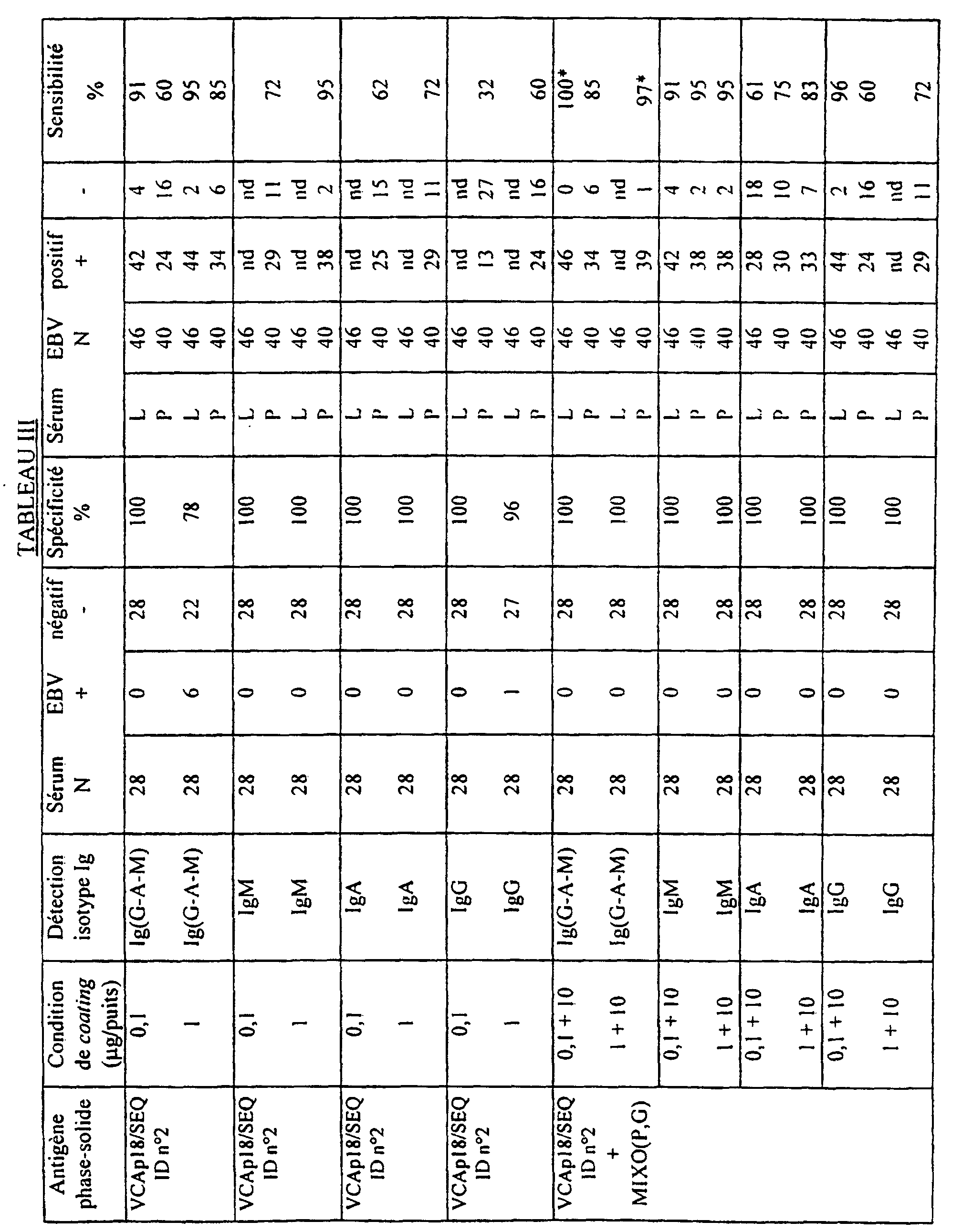

Ce Tableau montre les résultats obtenus pour 86 sérums de sujets porteurs sains ou de sujets primo-infectés lorsque le peptide VCAp18/SEQ ID n°2 est utilisé seul ; on observe pour ces deux populations respectivement 60 % et 51 % de réponses positives, sans perte de spécificité.

Légende du Tableau III : nd = non déterminé ; L = sérums de porteurs sains (IgM négatif et IgG positif) ; P = sérums de patients primoinfectés (IgM positif et IgG dans la plupart des cas positif). Il n'y a pas d'indication concernant les IgA avec les ELISA conventionnels.

Lorsqu'un réactif selon l'invention VCAp18/SEQ ID n°2 + MIXO(P,G) est utilisé, les pourcentages de résultats positifs obtenus dans les deux populations atteignent respectivement 97 % et 100 %. Ce dernier résultat (Tableau III, *) dépend des concentrations d'utilisation sélectionnées pour le peptide VCAp18/SEQ ID n°2 et concerne la réactivité globale IgG-A-M.

Si l'on observe l'ensemble des résultats, une bonne corrélation (98,8 %) est observée entre les méthodes classiques et la méthode mettant en oeuvre un réactif selon l'invention (bonne sensibilité).

Ceci démontre la pertinence de la sérodétection IgG-A-M, qui est habituellement considérée comme peu sensible et seulement IgG-spécifique.

En fait, avec les réactifs selon la présente invention, lorsque l'on compare les sensibilités individuelles. IgG, IgA et IgM avec les résultats obtenus avec la détection IgG-A-M selon l'invention, cette dernière correspond approximativement à la somme des résultats obtenus pour chaque isotype ; en particulier, aucun des sérums isotype-positifs n'a été négatif, lorsqu'une réactivité globale Ig(GAM) est détectée selon la méthode selon la présente invention.

Ces résultats montrent la spécificité et la sensibilité du réactif selon l'invention.

En outre, ces résultats montrent que les sérums des sujets ayant présenté une infection dans le passé présentent des taux résiduels d'IgM.

Les réactivités IgM sont surprenantes car contraires aux résultats observés avec les tests de l'Art antérieur.

Ces résultats suggèrent notamment que les IgM persistent avec les IgG pendant une longue période après l'infection.

## Revendications

1. Réactif de diagnostic d'une infection provoquée par un virus, **caractérisé en ce qu'**il comprend essentiellement un mélange constitué par (1) un fragment immunodominant d'une protéine dudit virus comprenant au plus 60 acides aminés, de préférence entre 20 et 30 acides aminés et (2) un mélange (dénommé mixotope) de peptides combinatoires convergents, dérivés dudit fragment immmunodominant, lesquels peptides sont obtenus par dégénération artificielle complète ou partielle dudit fragment immunodominant par remplacement systématique ou partiel de chaque amino acide par un autre selon une matrice de remplaçabilité convenable.

2. Réactif de diagnostic selon la revendication 1, **caractérisé en ce que** pour le diagnostic des infections à EBV, il comprend essentiellement un mélange constitué par (1) un fragment C-terminal de la protéine VCAp18 SEQ ID n°1 du virus d'Epstein-Barr (EBV) comprenant au plus 60 acides aminés, de préférence entre 20 et 30 acides aminés, et (2) un mélange ou mixotope de peptides combinatoires convergents, dérivés dudit fragment C-terminal.

3. Réactif selon la revendication 2, **caractérisé en ce que** ledit fragment C-terminal de la protéine VCAp18 est sélectionné dans le groupe constitué par les peptides VCAp18/SEQ ID n°2, VCAp18/SEQ ID n°3, VCAp18/SEQ ID n°4 et VCAp18/SEQ ID n°5.

4. Réactif selon la revendication 2 ou la revendication 3, **caractérisé en ce que** le mixotope correspond à une dégénération de l'ensemble de la séquence du peptide VCAp18 sélectionné.

5. Réactif selon la revendication 2 ou la revendication 3, **caractérisé en ce que** le mixotope correspond à une dégénération partielle de la séquence du peptide VCAp18 sélectionné.

6. Réactif selon la revendication 5, **caractérisé en ce que** lorsque ledit peptide est le peptide VCAp18/SEQ ID n°2, les résidus proline et/ou la séquence Gly ser Gly Gly Gly Gly (SEQ ID n°6) sont préférentiellement conservés.

7. Réactif selon la revendication 5, **caractérisé en ce que** lorsque ledit peptide est le peptide VCAp18/SEQ ID n°3, les résidus proline et/ou sérine et/ou alanine de séquences répétitives sont préférentiellement conservés.

8. Réactif selon la revendication 5, **caractérisé en ce que** lorsque ledit peptide est le peptide VCAp18/SEQ ID n°4, les résidus sérine et/ou proline et/ou alanine et/ou la séquence Gly ser Gly Gly Gly Gly (SEQ ID n°6) et/ou la séquence Ala Ala Ala Ser Ala Ala Ala Ala (SEQ ID n°7) du peptide VCAp18/SEQ ID n°4 sont préférentiellement conservés.

9. Réactif selon la revendication 5, **caractérisé en ce que** lorsque ledit peptide est le peptide VCAp18/SEQ ID n°5, les résidus sérine et/ou alanine et/ou la séquence Ala Ala Ala ser Ala Ala Ala Ala (SEQ ID n°7) du peptide VCAp18/SEQ ID n°5 sont préférentiellement conservés.

10. Réactif selon l'une quelconque de revendications 1 à 9, **caractérisé en ce que** ledit réactif est fixé sur un support solide, de préférence des plaques de microtitration.

11. Réactif selon l'une quelconque de revendications 2 à 10, **caractérisé en ce que** le rapport peptide C-terminal de la protéine SEQ ID n°1:mixotope dans le mélange est compris entre 1:10 et 1:100.

12. Procédé de diagnostic d'une infection virale, par une méthode immuno-enzymatique, **caractérisé en ce qu'**il met en oeuvre un réactif de diagnostic selon l'une quelconque des revendications 1 à 11.

13. Procédé de diagnostic selon la revendication 12, **caractérisé en ce que** pour le diagnostic d'une infection à EBV par une méthode immuno-enzymatique, il comprend :
- la mise en contact d'un sérum à analyser avec un réactif selon l'une quelconque des revendications 2 à 11,
- l'addition d'anticorps anti-Ig humaines, couplés à une enzyme et
- la révélation qualitative et/ou quantitative des anticorps anti-VCA éventuellement présents dans le sérum à analyser par addition du substrat de l'enzyme.

14. Procédé selon la revendication 13, **caractérisé en ce qu'**il comprend :
- la fixation d'un réactif selon l'une quelconque des revendications 2 à 11 sur un support, tel qu'une plaque de microtitration,
- l'addition du sérum à analyser et
- la détection de la fixation des anticorps anti-VCA présents dans ledit sérum par addition d'anticorps anti-Ig humaines (G-A-M), couplés à une enzyme et
- la révélation qualitative et/ou quantitative au spectrophotomètre par addition du substrat de l'enzyme.

15. Procédé de surveillance et de détection différentielle des stades d'une infection à EBV, par une méthode immuno-enzymatique, **caractérisé en ce qu'**il comprend :
- la fixation d'un réactif selon l'une quelconque des revendications 2 à 11 sur un support, tel qu'une microplaque de titration,
- l'addition du sérum à analyser,
- la détection de la fixation des anticorps anti-VCA, éventuellement présents dans ledit sérum, par addition d'anticorps anti-Ig humaines couplés à une enzyme, lesquels anticorps sont sélectionnés dans le groupe constitué par les anticorps anti-IgG humaines, les anticorps anti-IgM humaines et les anticorps anti-IgA humaines et
- la révélation qualitative ou quantitative au spectrophotomètre par addition du substrat de l'enzyme.

16. Procédé de détection précoce du cancer du nasopharynx par une méthode immuno-enzymatique, **caractérisé en ce qu'**il comprend :
- la fixation d'un réactif selon l'une quelconque des revendications 2 à 11 sur un support, tel qu'une microplaque de titration,
- l'addition du sérum à analyser,
- la détection de la fixation des anticorps anti-VCA, éventuellement présents dans ledit sérum, par addition d'anticorps anti-IgA humaines couplés à une enzyme, et
- la révélation qualitative ou quantitative au spectrophotomètre par addition du substrat de l'enzyme.

17. Kit ou coffret de diagnostic d'une infection virale, **caractérisé en ce qu'**il comprend au moins un réactif de diagnostic selon l'une quelconque des revendication 1 à 11.

## Patentansprüche

1. Reagens zur Diagnose einer durch ein Virus hervorgerufenen Infektion, **dadurch gekennzeichnet, dass** es im Wesentlichen ein Gemisch umfasst, bestehend aus (1) einem immundominanten Fragment eines Proteins des Virus, das höchstens 60 Aminosäuren, vorzugsweise zwischen 20 und 30 Aminosäuren, umfasst, und (2) einem Gemisch (als Mixotop bezeichnet) kombinatorischer, konvergenter Peptide, die von dem immundominanten Fragment abgeleitet sind, wobei die Peptide durch vollständige oder partielle künstliche Degeneration des immundominanten Fragments durch systematisches oder partielles Ersetzen jeder Aminosäure durch eine andere nach einer passenden Austauschbarkeitsmatrize erhalten wurden.

2. Reagens zur Diagnose nach Anspruch 1, **dadurch gekennzeichnet, dass** es für die Diagnose von Infektionen durch EBV im Wesentlichen ein Gemisch umfasst, bestehend aus (1) einem C-terminalen Fragment Proteins VCAp18, SEQ ID NO. 1 des Epstein-Barr-Virus (EBV), das höchstens 60 Aminosäuren, vorzugsweise zwischen 20 und 30 Aminosäuren, umfasst, und (2) einem Gemisch oder Mixotop kombinatorischer, konvergenter Peptide, die von dem C-terminalen Fragment abgeleitet sind.

3. Reagens nach Anspruch 2, **dadurch gekennzeichnet, dass** das C-terminale Fragment des Proteins VCAp18 aus der Gruppe ausgewählt ist, die aus den Peptiden VCAp18/SEQ ID NO. 2, VCAp18/SEQ ID NO. 3, VCAp18/SEQ ID NO. 4 und VCAp18/SEQ ID NO. 5 besteht.

4. Reagens nach Anspruch 2 oder Anspruch 3, **dadurch gekennzeichnet, dass** das Mixotop einer Degeneration der Gesamtheit der Sequenz des ausgewählten Peptids VCAp18 entspricht.

5. Reagens nach Anspruch 2 oder Anspruch 3, **dadurch gekennzeichnet, dass** das Mixotop einer partiellen Degeneration der Sequenz des ausgewählten Peptids VCAp18 entspricht.

6. Reagens nach Anspruch 5, **dadurch gekennzeichnet, dass**, wenn das Peptid das Peptid VCAp18/SEQ ID NO. 2 ist, die Prolinreste und/oder die Sequenz Gly Ser Gly Gly Gly Gly (SEQ ID NO. 6) vorzugsweise konserviert sind/ist.

7. Reagens nach Anspruch 5, **dadurch gekennzeichnet, dass**, wenn das Peptid das Peptid VCAp18/SEQ ID NO. 3 ist, die Prolin- und/oder Serin- und/oder Alanin-Reste der sich wiederholenden Sequenzen vorzugsweise konserviert sind.

8. Reagens nach Anspruch 5, **dadurch gekennzeichnet, dass**, wenn das Peptid das Peptid VCAp18/SEQ ID NO. 4 ist, die Serin- und/oder Prolin- und/oder Alanin-Reste und/oder die Sequenz Gly Ser Gly Gly Gly Gly (SEQ ID NO. 6) und/oder die Sequenz Ala Ala Ala Ser Ala Ala Ala Ala (SEQ ID NO. 7) des Peptids VCAp18/SEQ ID NO. 4 vorzugsweise konserviert sind.

9. Reagens nach Anspruch 5, **dadurch gekennzeichnet, dass**, wenn das Peptid das Peptid VCAp18/SEQ ID NO. 5 ist, vorzugsweise die Serin- und/oder Alanin-Reste und/oder die Sequenz Ala Ala Ala Ser Ala Ala Ala Ala (SEQ ID NO. 7) des Peptids VCAp18/SEQ ID NO. 5, konserviert sind.

10. Reagens nach einem der Ansprüche 1 bis 9, **dadurch gekennzeichnet, dass** das Reagens an einem festen Träger, vorzugsweise Mikrotiterplatten, fixiert ist.

11. Reagens nach einem der Ansprüche 2 bis 10, **dadurch gekennzeichnet, dass** das Verhältnis C-terminales Peptid des Proteins SEQ ID NO. 1: Mixotop in dem Gemisch zwischen 1:10 und 1:100 liegt.

12. Verfahren zur Diagnose einer viralen Infektion durch ein immunenzymatisches Verfahren, **dadurch gekennzeichnet, dass** es ein Reagens zur Diagnose nach einem der Ansprüche 1 bis 11 verwendet.

13. Verfahren zur Diagnose nach Anspruch 12, **dadurch gekennzeichnet, dass** es zur Diagnose einer Infektion mit EBV durch ein immunenzymatisches Verfahren umfasst:
- In-Kontakt-bringen eines zu analysierenden Serums mit einem Reagens nach einem der Ansprüche 2 bis 11,
- Zusatz von humanen anti-Ig-Antikörpern, die an ein Enzym gebunden sind, und
- qualitative und/oder quantitative Entwicklung von anti-VCA-Antikörpern, die ggf. in dem zu analysierenden Serum vorliegen, durch Zusatz des Substrats des Enzyms.

14. Verfahren nach Anspruch 13, **dadurch gekennzeichnet, dass** es umfasst:
- die Fixierung eines Reagens nach einem der Ansprüche 2 bis 11 auf einem Träger wie einer Mikrotierplatte,
- Zusatz des zu analysierenden Serums und
- Detektion der Fixierung der anti-VCA-Antikörper, die in dem Serum vorliegen, durch Zusatz von humanen anti-Ig-Antikörpern (G-A-M), die an ein Enzym gebunden sind, und
- qualitative und/oder quantitative Entwicklung durch Zusatz des Substrats des Enzyms mittels Spektralphotometer.

15. Verfahren zur Überwachung und differenziellen Detektion der Stadien einer Infektion durch EBV durch ein immunenzymatisches Verfahren, **dadurch gekennzeichnet, dass** es umfasst:
- Fixierung eines Reagens nach einem der Ansprüche 2 bis 11 auf einem Träger wie einer Mikrotiterplatte,
- Zusatz des zu analysierenden Serums,
- Detektion der Fixierung der anti-VCA-Antikörper, die ggf. in dem Serum vorliegen, durch Zusatz von humanen anti-Ig-Antikörpern, die an ein Enzym gebunden sind, wobei die Antikörper aus der Gruppe bestehend aus humanen anti-IgG-Antikörpern, humanen anti-IgM-Antikörpern und humanen anti-IgA-Antikörpern ausgewählt sind, und
- qualitative oder quantitative Entwicklung durch Zusatz des Substrates des Enzyms mittels Spektralphotometer.

16. Verfahren zur frühen Detektion von Krebs des Nasopharynx durch ein immunenzymatisches Verfahren, **dadurch gekennzeichnet, dass** es umfasst:
- Fixierung eines Reagens nach einem der Ansprüche 2 bis 11 auf einem Träger wie einer Mikrotiterplatte,
- Zusatz des zu analysierenden Serums,
- Detektion der Fixierung der anti-VCA-Antikörper, die ggf. in dem Serum vorliegen, durch Zusatz von humanen anti-IgA-Antikörpern, die an ein Enzym gebunden sind, und
- qualitative oder quantitative Entwicklung durch Zusatz des Substrates des Enzyms mittels Spektralphotometer.

17. Kit oder Kassette zur Diagnose einer viralen Infektion, **dadurch gekennzeichnet, dass** er/sie mindestens ein Reagens zur Diagnose nach einem der Ansprüche 1 bis 11 umfasst.

## Claims

1. Reagent for the diagnosis of an infection caused by a virus, **characterized in that** it comprises essentially a mixture consisting of (1) an immunodominant fragment of a protein of the said virus comprising at most 60 amino acids, preferably between 20 and 30 amino acids and (2) a mixture (called mixotope) of convergent combinatory peptides derived from the said immunodominant fragment, which peptides are obtained by complete or partial artificial degeneration of the said immunodominant fragment by systematic or partial replacement of each amino acid with another according to a suitable replaceability matrix.

2. Diagnostic reagent according to Claim 1, **characterized in that** for the diagnosis of EBV infections, it comprises essentially a mixture consisting of (1) a C-terminal fragment of the protein VCAp18 SEQ ID No. 1, of the Epstein-Barr virus (EBV) comprising at most 60 amino acids, preferably between 20 and 30 amino acids and (2) a mixture or mixotope of convergent combinatory peptides derived from the said C-terminal fragment.

3. Reagent according to Claim 2, **characterized in that** the said C-terminal fragment of the VCAp18 protein is selected from the group consisting of the peptides VCAp18/SEQ ID No. 2, VCAp18/SEQ ID No. 3, VCAp18/SEQ ID No. 4 and VCAp18/SEQ ID No. 5.

4. Reagent according to Claim 2 or Claim 3, **characterized in that** the mixotope corresponds to a degeneration of the entire sequence of the selected peptide VCAp18.

5. Reagent according to Claim 2 or Claim 3, **characterized in that** the mixotope corresponds to a partial degeneration of the sequence of the selected peptide VCAp18.

6. Reagent according to Claim 5, **characterized in that** when the said peptide is the peptide VCAp18/SEQ ID No. 2, the proline residues and/or the sequence Gly Ser Gly Gly Gly Gly (SEQ ID No. 6) are preferentially conserved.

7. Reagent according to Claim 5, **characterized in that** when the said peptide is the peptide VCAp18/SEQ ID No. 3, the proline and/or serine and/or alanine residues of the repetitive sequences are preferentially conserved.

8. Reagent according to Claim 5, **characterized in that** when the said peptide is the peptide VCAp18/SEQ ID No. 4, the serine and/or proline and/or alanine residues and/or the sequence Gly Ser Gly Gly Gly Gly (SEQ ID No. 6) and/or the sequence Ala Ala Ala Ser Ala Ala Ala Ala (SEQ ID No. 7) of the peptide VCAp18/SEQ ID No. 4 are preferentially conserved

9. Reagent according to Claim 5, **characterized in that** when the said peptide is the peptide VCAp18/SEQ ID No. 5, the serine and/or alanine residues and/or the sequence Ala Ala Ala Ser Ala Ala Ala Ala (SEQ ID No. 7) of the peptide VCAp18/SEQ ID No. 5 are preferentially conserved.

10. Reagent according to any one of Claims 1 to 9, **characterized in that** the said reagent is attached to a solid support, preferably microtitre plates.

11. Reagent according to any one of Claims 2 to 10, **characterized in that** the C-terminal peptide of the protein SEQ ID No. 1:mixotope ratio in the mixture is between 1:10 and 1:100.

12. Method for the diagnosis of a viral infection, by an immunoenzymatic method, **characterized in that** it uses a diagnostic reagent according to any one of Claims 1 to 11.

13. Diagnostic method according to Claim 12, **characterized in that**, for the diagnosis of an EBV infection by an immunoenzymatic method, it comprises:
- bringing a serum to be analysed into contact with a reagent according to any one of Claims 2 to 11,
- the addition of anti-human Ig antibodies coupled to an enzyme, and
- the qualitative and/or quantitative revealing of the anti-VCA antibodies which may be present in the serum to be analysed by addition of the enzyme substrate.

14. Method according to Claim 13, **characterized in that** it comprises:
- the attachment of a reagent according to any one of Claims 2 to 11 onto a support, such as a microtitre plate,
- the addition of the serum to be analysed, and
- the detection of the attachment of the anti-VCA antibodies present in the said serum by addition of anti-human Ig (G-A-M) antibodies coupled to an enzyme, and
- the qualitative and/or quantitative revealing in a spectrophotometer by addition of the enzyme substrate.

15. Method for the surveillance and differential detection of the stages of an EBV infection by an immunoenzymatic method, **characterized in that** it comprises:
- the attachment of a reagent according to any one of Claims 2 to 11 onto a support, such as a microtitre plate,
- the addition of the serum to be analysed,
- the detection of the attachment of the anti-VGA antibodies, which may be present in the said serum, by the addition of anti-human Ig antibodies coupled to an enzyme, which antibodies are selected from the group consisting of the anti-human IgG antibodies, the anti-human IgM antibodies and the anti-human IgA antibodies, and
- the qualitative or quantitative revealing in a spectrophotometer by the addition of the enzyme substrate.

16. Method for the early detection of the cancer of the nasopharynx by an immunoenzymatic method, **characterized in that** it comprises:
- the attachment of a reagent according to any one of Claims 2 to 11 onto a support, such as a microtitre plate,
- the addition of the serum to be analysed,
- the detection of the attachment of the anti-VCA antibodies, which may be present in the said serum, by the addition of anti-human IgA antibodies coupled to an enzyme, and
- the qualitative or quantitative revealing in a spectrophotometer by the addition of the enzyme substrate.

17. Kit or box for the diagnosis of a viral infection, **characterized in that** it comprises at least one diagnostic reagent according to any one of Claims 1 to 11.


### LISTE DE SEQUENCES

(1) INFORMATIONS GENERALES:
   (i) DEPOSANT:
      (A) NOM: INSTITUT PASTEUR DE LILLE
      (B) RUE: 1 RUE DU PROFESSEUR CALMETTE
      (C) VILLE: LILLE
      (E) PAYS: FRANCE
      (F) CODE POSTAL: 59019 CEDEX

      (A) NOM: CENTRE NATIONAL DE LA RECHERCHE SCIENTIFIQUE - CNRS
      (B) RUE: 3 RUE MICHEL-ANGE
      (C) VILLE: PARIS
      (E) PAYS: FRANCE
      (F) CODE POSTAL: 75794 CEDEX 16

      (A) NOM: TRANCHAND BUNEL DENIS
      (B) RUE: 2 RUE JACQUES BREL
      (C) VILLE: RONCHIN
      (E) PAYS: FRANCE
      (F) CODE POSTAL: 59790

      (A) NOM: GRAS MASSE HELENE
      (B) RUE: 321 RUE DE LA ROSIERE
      (C) VILLE: MERIGNIES
      (E) PAYS: FRANCE
      (F) CODE POSTAL: 59710

      (A) NOM: AURIAULT CLAUDE
      (B) RUE: 60 RUE LOUIS GAISLAIN
      (C) VILLE: NOMAIN
      (E) PAYS: FRANCE
      (F) CODE POSTAL: 59310

      (A) NOM: TARTAR ANDRE
      (B) RUE: 1 RUE DU MOULIN
      (C) VILLE: VITRY EN ARTOIS
      (E) PAYS: FRANCE
      (F) CODE POSTAL: 62490

      (A) NOM: DIESIS ERIC
      (B) RUE: 12 RUE DU CAPITAINE HAZEBROUCK
      (C) VILLE: HAUBOURDIN
      (E) PAYS: FRANCE
      (F) CODE POSTAL: 59320

      (A) NOM: BOUREZ BRIGITTE
      (B) RUE: 7 RUE DU CAPITAINE PICAVET
      (C) VILLE: LEERS
      (E) PAYS: FRANCE
      (F) CODE POSTAL: 59115
   (ii) TITRE DE L'INVENTION: REACTIF DE DETECTION ET DE SUIVI DES INFECTIONS VIRALES ET SES APPLICATIONS.
   (iii) NOMBRE DE SEQUENCES: 7
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: Pacentin Release #1.0, Version #1.30 (OEB)
   (vi) DONNEES DE LA DEMANDE ANTERIEURE:
      (A) NUMERO DE LA DEMANDE: FR 97 08537
      (B) DATE DE DEPOT: 04-JUL-1997
(2) INFORMATIONS POUR LA SEQ ID NO: 1:
   (i) CARACTERISTIQUES DE LA SEQUENCE:
      (A) LONGUEUR: 176 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 1:
   Met Ala Arg Arg Leu Pro Lys Pro Thr Leu Gln Gly Arg Leu Glu Ala
   1               5                   10                  15
   Asp Phe Pro Asp Ser Pro Leu Leu Pro Lys Phe Gln Glu Leu Asn Gln
               20                  25                  30
   Asn Asn Leu Pro Asn Asp Val Phe Arg Glu Ala Gln Arg Ser Tyr Leu
           35                  40                  45
   Val Phe Leu Thr Ser Gln Phe Cys Tyr Glu Glu Tyr Val Gln Arg Thr
       50                  55                  60
   Phe Gly Val Pro Arg Arg Gln Arg Ala Ile Asp Lys Arg Gln Arg Ala
   65                  70                  75                  80
   Ser Val Ala Gly Ala Gly Ala His Ala His Leu Gly Gly Ser Ser Ala
                   85                  90                  95
   Thr Pro Val Gln Gln Ala Gln Ala Ala Ala Ser Ala Gly Thr Gly Ala
               100                 105                 110
   Leu Ala Ser Ser Ala Pro Ser Thr Ala Val Ala Gln Ser Ala Thr Pro
           115                 120                 125
   Ser Val Ser Ser Ser Ile Ser Ser Leu Arg Ala Ala Thr Ser Gly Ala
       130                 135                 140
   Thr Ala Ala Ala Ser Ala Ala Ala Ala Val Asp Thr Gly Ser Gly Gly
   145                 150                 155                 160
   Gly Gly Gln Pro His Asp Thr Ala Pro Arg Gly Ala Arg Lys Lys Gln
                   165                 170                 175
(2) INFORMATIONS POUR LA SEQ ID NO: 2:
   (i) CARACTERISTIQUES DE LA SEQUENCE:
      (A) LONGUEUR: 71 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 2:
   Xaa Ala Leu Ala Val Ala Leu Ala Ser Pro Thr His Arg Gly Leu Tyr
   1               5                   10                  15
   Ser Glu Arg Gly Leu Tyr Gly Leu Tyr Gly Leu Tyr Gly Leu Tyr Gly
               20                  25                  30
   Leu Asn Pro Arg His Ile Ser Ala Ser Pro Thr His Arg Ala Leu Ala
           35                  40                  45
   Pro Arg Ala Arg Gly Gly Leu Tyr Ala Leu Ala Ala Arg Gly Leu Tyr
       50                  55                  60
   Ser Leu Tyr Ser Gly Leu Asn
   65                  70
(2) INFORMATIONS POUR LA SEQ ID NO: 3:
   (i) CARACTERISTIQUES DE LA SEQUENCE:
      (A) LONGUEUR: 30 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 3:
   Ser Thr Ala Val Ala Gln Ser Ala Thr Pro Ser Val Ser Ser Ser Ile
   1               5                   10                  15
   Ser Ser Leu Arg Ala Ala Thr Ser Gly Ala Thr Ala Ala Ala
               20                  25                  30
(2) INFORMATIONS POUR LA SEQ ID NO: 4:
   (i) CARACTERISTIQUES DE LA SEQUENCE:
      (A) LONGUEUR: 58 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 4:
   Ser Thr Ala Val Ala Gln Ser Ala Thr Pro Ser Val Ser Ser Ser Ile
   1 5 10 15
   Ser Ser Leu Arg Ala Ala Thr Ser Gly Ala Thr Ala Ala Ala Ser Ala
   20 25 30
   Ala Ala Ala Val Asp Thr Gly Ser Gly Gly Gly Gly Gln Pro His Asp
   35 40 45
   Thr Ala Pro Arg Gly Ala Arg Lys Lys Gln
   50 55
(2) INFORMATIONS POUR LA SEQ ID NO: 5:
   (i) CARACTERISTIQUES DE LA SEQUENCE:
      (A) LONGUEUR: 30 acides aminés
      (B) TYPE: acide aminé
      (C) NOMBRE DE BRINS: simple
      (D) CONFIGURATION: linéaire
   (ii) TYPE DE MOLECULE: protéine
   (xi) DESCRIPTION DE LA SEQUENCE: SEQ ID NO: 5:
   Ser Val Ser Ser Ser Ile Ser Ser Leu Arg Ala Ala Thr Ser Gly Ala
   1 5 10 15
   Thr Ala Ala Ala Ser Ala Ala Ala Ala Val Asp Thr Gly Ser
   20 25 30
(2) INFORMATIONS POUR LA SEQ ID NO: 6:
   (i) CARACTERISTIQUES DE LA SEQUENCE:
      (A) LONGUEUR: 6 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 6:
   Gly Ser Gly Gly Gly Gly
   1 5
(2) INFORMATIONS POUR LA SEQ ID NO: 7:
   (i) CARACTERISTIQUES DE LA SEQUENCE:
      (A) LONGUEUR: 8 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 7:
   Ala Ala Ala Ser Ala Ala Ala Ala
   1               5